(19) Europäisches Patentamt / European Patent Office / Office européen des brevets

(11) **EP 2 011 481 B1**

(12) **FASCICULE DE BREVET EUROPEEN**

(45) Date de publication et mention de la délivrance du brevet:
**16.03.2016 Bulletin 2016/11**

(51) Int Cl.:
*A61K 8/72* *(2006.01)*      *A61K 8/81* *(2006.01)*
*A61Q 17/04* *(2006.01)*      *A61K 8/11* *(2006.01)*

(21) Numéro de dépôt: **08158698.4**

(22) Date de dépôt: **20.06.2008**

(54) **Composition de protection solaire contenant l'association d'un polymère semi-cristallin et de particules de latex creuses**

Sonnenschutzzusammensetzung, die eine Verbindung aus einem halbkristallinen Polymer und hohle Latexpartikeln enthält

Sun-protection composition containing the association of a semi-crystalline polymer and hollow latex particles

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **06.07.2007 FR 0756322**

(43) Date de publication de la demande:
**07.01.2009 Bulletin 2009/02**

(73) Titulaire: **L'Oréal**
**75008 Paris (FR)**

(72) Inventeurs:
• **Candau, Didier**
  **91570, Bievres (FR)**
• **Boutelet, Karl**
  **75011, Paris (FR)**

(74) Mandataire: **Miszputen, Laurent**
  **L'Oréal**
  **D.I.P.I.**
  **25-29 Quai Aulagnier**
  **92600 Asnières (FR)**

(56) Documents cités:
EP-A- 0 669 124      EP-A- 0 893 119
EP-A- 1 092 421      EP-A- 1 331 000
WO-A-2006/048159

EP 2 011 481 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente demande se rapporte à une composition cosmétique ou dermatologique comprenant au moins un filtre UV organique et/ou un filtre inorganique, caractérisée par le fait qu'elle contient, ans un milieu physiologiquement acceptable au moins l'association (A) et (B) suivante :

A) un polymère semi-cristallin solide à température ambiante et ayant une température de fusion supérieure ou égale à 30°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre Mn supérieure ou égale à 1000 ; **ledit polymère étant choisi parmi les polymères issus d'un monomère à chaîne cristallisable choisi parmi les (méth)acrylates d'alkyle saturés en $C_{14}$ à $C_{22}$ et**

B) des particules de latex creuses **constituées d'un copolymère de styrène et d'acide (méth)acrylique ou l'un de ses ($C_1$-$C_{20}$)alkyl esters** ayant une taille de particule allant de 150 à 380 nm.

L'invention concerne également l'utilisation cosmétique de ladite association,, dans une composition à application topique destinée à la protection de la peau, des lèvres, des ongles, des cheveux, du cuir chevelu, des cils ou des sourcils, ongles contre les rayonnements UV" en particulier le rayonnement solaire, contenant au moins un filtre UV organique et/ou un filtre UV inorganique, comme agent permettant d'augmenter le facteur de protection solaire (SPF).

On sait que les rayons lumineux de longueurs d'onde comprises entre 280 nm et 400 nm permettent le brunissement de l'épiderme humain et que, par ailleurs, les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent souvent sous la forme d'une émulsion de type huile-dans-eau, ou eau-dans-huile, de gels ou de produits anhydres qui contiennent, à des concentrations diverses, un ou plusieurs filtres organiques et/ou inorganiques, insolubles et/ou liposolubles et/ou hydrosolubles capables d'absorber sélectivement le rayonnement UV nocif. Ces filtres et leurs quantités étant sélectionnés en fonction de l'indice de protection recherché. Selon leur caractère lipophile ou au contraire hydrophile, ces filtres peuvent se répartir, respectivement, soit dans la phase grasse, soit dans la phase aqueuse, de la composition finale.

Pour des raisons de stabilité de ces compositions et/ou de tolérance sur la peau les cheveux, ou les muqueuses, il parfois difficile d'utiliser de fortes quantités de filtres chimiques et/ou de filtres minéraux pour augmenter l'indice de protection des compositions solaires. Aussi la demanderesse a cherché un autre moyen pour augmenter l'indice de protection de ces compositions. 1.

L'efficacité des filtres chimiques ou physiques est souvent limitée par les problèmes de dispersion de ces filtres à partir des compositions cosmétiques. Ceci a pour conséquence de provoquer une hétérogénéité du film formé à la surface de la peau qui nuit à la qualité, stabilité et efficacité des produits solaires. On a déjà proposé dans le brevet US 5,663,213, les demandes EP11092421, EP1281,388, EP1281389, EP1291390, DE10138499 d'utiliser des particules de latex creuses dans des formulations solaires dans le but d'augmenter leur indice de protection.

On a également proposé dans la demande EP1331000 d'utiliser un polymère semi-cristallin dans des formulations solaires dans le but d'augmenter leur indice de protection.

De façon surprenante, la demanderesse a trouvé qu'en combinant des filtres UV avec une association constituée d'au moins un polymère semi-cristallin et de particules de latex creuses, on obtenait une synergie au niveau du pouvoir filtrant des filtres et, de ce fait, une nette amélioration de l'indice de protection (SPF ou IP) de la composition les contenant par rapport à une composition contenant le ou les mêmes filtres et le polymère semi-cristallin seul et par rapport à un composition ou les mêmes filtres avec les particules de latex creuses seules. Les composions selon l'invention présentent de bonnes propriétés cosmétiques ainsi qu'une bonne rémanence à l'eau.

La présente demande se rapporte donc à une composition cosmétique ou dermatologique comprenant au moins un filtre UV organique et/ou au moins un filtre inorganique, caractérisée par le fait qu'elle contient, dans un milieu physiologiquement acceptable, au moins l'association (A) et (B) suivante :

A) au moins un polymère semi-cristallin solide à température ambiante et ayant une température de fusion supérieure ou égale à 30°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 1000 à1000 ; **ledit polymère étant choisi parmi les polymères issus d'un monomère à chaîne cristallisable choisi parmi les (méth)acrylates d'alkyle saturés en $C_{14}$ à $C_{22}$ et**

B) des particules de latex creuses **constituées d'un copolymère de styrène et d'acide (méth)acrylique ou l'un de ses $(C_1-C_{20})$alkyl esters** ayant une taille de particule allant de 150 à 380 nm.

L'invention concerne également l'utilisation cosmétique de ladite association, dans une composition à application topique destinée à la protection de la peau et/ou des cheveux contre les rayonnements UV, en particulier le rayonnement solaire, contenant, dans un milieu physiologiquement acceptable au moins un filtre UV organique et/ou un filtre UV minéral, comme agent permettant d'augmenter le facteur de protection solaire (SPF).

On entend dans la présente demande par « température ambiante » une température de 25°C.

On entend par «milieu physiologiquement acceptable», un milieu non toxique et susceptible d'être appliqué sur la peau, les lèvres, les cheveux, le cuir chevelu, les cils, les sourcils les ongles ou toute autre zone cutanée du corps. La composition de l'invention peut constituer notamment une composition cosmétique ou dermatologique.

On entend par « latex », des particules de polymère sous forme de dispersion aqueuse généralement stabilisée par au moins un agent émulsionnant.

La composition peut constituer notamment une composition cosmétique. De manière générale, une composition cosmétique est destinée à être mise en contact avec les parties superficielles du corps humain. Une composition cosmétique solaire permet de lutter contre les effets des rayons UV sur les couches superficielles de la peau et notamment contre les effets sur le vieillissement de la peau (rides et ridules).

Le polymère semi-cristallin utilisé dans la composition de l'invention permet d'obtenir des indices de protection plus élevés sans augmenter le taux de filtres chimiques, donc d'améliorer l'indice de protection pour une quantité de filtre déterminée. Il est généralement introduit dans la phase grasse liquide (appelée aussi ci-après phase huileuse).

Par "polymères", on entend au sens de l'invention des composés comportant au moins 2 motifs de répétition, de préférence au moins 3 motifs de répétition et plus spécialement au moins 10 motifs de répétition.

Par "polymère semi-cristallin", on entend au sens de l'invention, des polymères comportant une partie cristallisable, chaîne pendante cristallisable ou séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe.

Par "séquence", on entend généralement au moins 5 motifs de répétition identiques. La ou les séquences cristallisables sont alors de nature chimique différente de la ou des séquences amorphes.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère. Avantageusement, la "chaîne pendante cristallisable" peut être une chaîne comportant au moins 6 atomes de carbone.

POLYMERES SEMI-CRISTALLINS

Le polymère semi-cristallin selon l'invention a une température de fusion supérieure ou égale à 30 °C, de préférence allant de 30 °C à 80 °C, et plus préférentiellement allant de 30 °C à 70 °C. Cette température de fusion est une température de changement d'état du premier ordre. Cette température de fusion peut être mesurée par toute méthode connue et en particulier à l'aide d'un calorimètre à balayage différentiel (D.S.C).

De façon avantageuse, le ou les polymères semi-cristallins auxquels s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

Selon un mode plus particulier de réalisation de l'invention, le **polymère semi-cristallin** est plus particulièrement choisi parmi les poly(acrylate de stéaryle) ou les poly(acrylate de béhényle). Plus particulièrement, on choisira le produit Intelimer® IPA 13-1 de la société Landec, qui est un polyacrylate de stéaryle de poids moléculaire d'environ 145 000 et dont la température de fusion est égale à 49°C.

La quantité de polymère semi-cristallin dans la composition de l'invention peut varier dans une large mesure suivant le but recherché. La quantité de polymères semi-cristallins peut aller par exemple de 0,1 à 50 % en poids de matière active, de préférence de 0,5 à 20 % en poids de matière active et mieux de 1 à 10 % en poids de matière active par rapport au poids total de la composition.

PARTICULES DE LATEX CREUSES

Les particules creuses de latex conformes à l'invention **selon la revendication 1** ont une taille de particule allant généralement de 100 à 380 nm et de préférence de 150 à 375 nm et plus préférentiellement de 190 à 350 nm et plus particulièrement de 251 à 325 nm ; la taille de particule étant mesurée par un spectrophotomètre à corrélation de photons BROOKHAVEN BI-90.

Pour une taille de particule donnée, les particules de latex selon l'invention doivent en général posséder une fraction creuse maximale. De préférence, les particules de latex contiennent une fraction vide de 0,1 à 50% et plus préférentiellement de 5 à 50%. Les fractions vides sont déterminées en comparant le volume occupée par les particules de latex après avoir été compactées à partir d'une dispersion diluée dans une centrifugeuse par rapport au volume de particules non vides dans la même composition.

Les particules de latex creuses selon l'invention peuvent être obtenues à partir de particules comprenant au moins un polymère pour le coeur et au moins un polymère pour l'enveloppe. Le polymère de coeur et le polymère d'enveloppe peuvent être obtenus a partir d'une seule étape de polymérisation ou par une séquence des étapes de polymérisation.

Les particules de latex creuses selon l'invention peuvent être préparées par les techniques classiques de polymérisation en émulsion. De tels procédés sont notamment décrits dans les brevets US4427836 ; US4469825 ; US4594363 ; US4677003 ; US4920160 ; US4970241 ; ou par les techniques classiques de polymérisation décrites dans les demandes et brevets suivants : EP267726 ; EP331421 ; US490229 ; US5157084.

Les monomères utilisés pour l'enveloppe des particules de latex sont de préférence constitués d'une ou plusieurs unités éthyléniques non-ioniques insaturées. De façon optionnelle, un ou plusieurs monomères monoéthyléniquement insaturés contenant au moins un groupe acide carboxylique peuvent être polymérisés dans l'enveloppe.

Les monomères constituant l'enveloppe sont sélectionnés de façon à ce qu'ils présentent une température de transition vitreuse (Tg) suffisamment élevée pour supporter le vide de la particule creuse de latex. De préférence, la température te transition vitreuse est supérieure à 50°C, plus préférentiellement supérieur à 60°C et encore plus préférentiellement supérieur à 70°C. Cette température Tg peut être déterminée par DSC (Differential Scanning Calorimetry).

Les monomères utilisés dans la polymérisation en émulsion du polymère de coeur des particules de latex de l'invention sont de préférence constitués d'un ou plusieurs monomères monoéthyléniquement insaturés contenant au moins un groupe acide carboxylique. De préférence, le coeur comprend au moins 5% en poids de monomère monoéthyléniquement insaturé contenant au moins un groupe acide carboxylique par rapport au poids total des monomères de coeur.

Le polymère de coeur peut par exemple être obtenu par homopolymérisation en émulsion du monomère monoéthyléniquement insaturé contenant au moins un groupe acide ou par copolymérisation de deux ou trois monomères monoéthyléniquement insaturés contenant au moins un groupe acide. De préférence, le monomère monoéthylénique insaturé contenant au moins un groupe acide est copolymérisé avec un ou plusieurs monomères non-ioniques éthyléniquement insaturés.

Le polymère de coeur ou le polymère de l'enveloppe peuvent contenir de 0,1% à 20% en poids, de préférence de 0,1 à 3% en poids de monomères polyéthyléniquement insaturés comme l'éthylèneglycol di(méth)acrylate, l'allyl(méth)acrylate, le di-1,3-butanol di(méth)acrylate, le diéthylèneglycol di(méth)acrylate, le trimethylolpropane tri(méth)acrylate ou le divinylbenzène. par rapport au poids total de monomères de coeur. Alternativement, le polymère de coeur ou le polymère d'enveloppe peuvent éventuellement contenir de 0,1 à 60% en poids de butadiène par rapport au poids total de monomères de coeur.

La partie vide du coeur des particules de latex est de préférence produite en gonflant le coeur par un agent gonflant comprenant un ou plusieurs composés volatils. L'agent pénètre l'enveloppe pour gonfler le coeur. Les composants volatils de l'agent gonflant peuvent être ensuite éliminés en séchant les particules de latex, créant ainsi un vide dans lesdites particules. De préférence, l'agent est une base aqueuse. On peut citer par exemple l'ammoniac, l'hydroxyde d'ammonium, les hydroxydes de métal alcalin comme l'hydroxyde de sodium, les amines volatiles comme le la triméthylamine ou la triéthylamine.

Les particules de latex creuses peuvent être introduites dans la composition de l'invention avec l'agent gonflant. Dans ce cas, les composés volatils sont éliminés au séchage de la composition. Les particules de latex creuses peuvent également être rajoutées à la composition après élimination des composés volatils de l'agent gonflant.

Les particules de latex creuses utilisables selon l'invention sont celles décrites dans le brevet US5,663,213 et la demande EP1092421.

Selon un mode particulier de l'invention, on utilisera des particules creuses de latex constituées d'un copolymère de styrène et d'acide (méth)acrylique ou l'une de ses ($C_1$-$C_{20}$)alkyl esters sous le nom INCI: Styrene/Acrylates Copolymer comme le produit vendu sous la dénomination commerciale Sunsphères Powder par la société ROHM & HAAS qui est une dispersion aqueuse à 86% de copolymère Styrene/Acrylates Copolymer dans un mélange de 11% de PEG-8 LAURATE , de 2,5% eau et de 0,5% de SODIUM DODECYLBENZENESULFONATE.

Les particules creuses de latex conformes à l'invention sont de préférence présentes dans la composition de l'invention dans des quantités allant de 0,1 à 20% en poids et plus préférentiellement de 0,5 à 10% en poids par rapport au poids total de la composition.

FILTRES UV ORGANIQUES (OU FILTRES SOLAIRES)

La composition de l'invention contient au moins un filtre UV minéral et/ou au moins un filtre UV organique

Les filtres UV organiques sont choisis parmi les filtres organiques hydrosolubles, les filtres organiques liposolubles ou insolubles dans les solvants couramment utilisés dans les produits solaires et leurs mélanges.

Les filtres UV organiques sont notamment choisis parmi les dérivés cinnamiques ; les anthranilates ; les dérivés salicyliques, les dérivés de dibenzoylméthane, les diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 ; les dérivés de mérocyanine tels que ceux décrits dans les demandes WO04/006878, WO05/058269 et WO06/032741 et leurs mélanges.

Comme exemples d'agents photoprotecteurs organiques complémentaires, on peut citer ceux désignés ci-dessous sous leur nom INCI :

Dérivés cinnamiques :

Ethylhexyl Methoxycinnamate vendu notamment sous le nom commercial PARSOL MCX par HOFFMANN LAROCHE,
Isopropyl Methoxycinnamate
Isoamyl Methoxycinnamate vendu sous le nom commercial NEO HELIOPAN E 1000 par HAARMANN ET REIMER,
DEA Methoxycinnamate,
Diisopropyl Methylcinnamate,
Glyceryl Ethylhexanoate Dimethoxycinnamate

Dérivés de dibenzoylméthane :

Butyl Methoxydibenzoylmethane vendu notamment sous le nom commercial PARSOL 1789 par HOFFMANN LAROCHE,
Isopropyl Dibenzoylmethane.

Dérivés de l'acide para-aminobenzoique :

PABA,
Ethyl PABA,
Ethyl Dihydroxypropyl PABA,

Ethylhexyl Diméthyl PABA vendu notamment sous le nom « ESCALOL 507 » par ISP,
Glyceryl PABA,
PEG-25 PABA vendu sous le nom « UVINUL P25 » par BASF,

Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

Dérivés de β,β-diphénylacrylate:

Octocrylene vendu notamment sous le nom commercial « UVINUL N539 » par BASF,
Etocrylene, vendu notamment sous le nom commercial « UVINUL N35 » par BASF,

Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12 2-(4-diethylamino-2-hydroxybenzoyl)-benzoate de n-hexyle vendu sous le nom commercial « UVINUL A+ » par BASF.

Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial « NEO HELIOPAN AP » par Haarmann et REIMER,

Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100» par FAIRMOUNT CHEMICAL ou sous forme micronisée en dispersion aqueuse sous le nom commercial « TINOSORB M » par CIBA SPECIALTY CHEMICALS,

Dérivés de triazine :

- Bis-Ethylhexyloxyphenol Methoxyphenyl Triazine vendu sous le nom commercial «TINOSORB S » par CIBA GEIGY,
- Ethylhexyl triazone vendu notamment sous le nom commerciale «UVINUL T150 » par BASF,
- Diethylhexyl Butamido Triazone vendu sous le nom commercial « UVASORB HEB » par SIGMA 3V,
- la 2,4,6-tris(4'-amino benzalmalonate de dinéopentyle)-s-triazine

- la 2,4,6-tris-(4'-amino benzalmalonate de diisobutyle)-s- triazine,
- la 2,4bis (4'-aminobenzoate de n-butyle)-6-(aminopropyltrisiloxane)-s-triazine,
- la 2,4bis (4'-aminobenzalmalonate de dinéopentyle)-6-(4'-aminobenzoate de n-butyle)-s-triazine,
- les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, WO2006/034992, WO2006/034985.•

Dérivés anthraniliques :

Menthyl anthranilate vendu sous le nom commercial commercial « NEO HELIOPAN MA » par Haarmann et REIMER,

Dérivés d'imidazolines :

Ethylhexyl Dimethoxybenzylidene Dioxoimidazoline Propionate,

Dérivés du benzalmalonate :

Polyorganosiloxane à fonctions benzalmalonate comme le Polysilicone-15 vendu sous la dénomination commerciale « PARSOL SLX » par HOFFMANN LA ROCHE

Dérivés de 4,4-diarylbutadiène :

-1,1-dicarboxy (2,2'-diméthyl-propyl)-4,4-diphénylbutadiène

Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

Les filtres organiques préférentiels sont choisis parmi

Ethylhexyl Methoxycinnamate
Ethylhexyl Salicylate,
Homosalate,
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Bis-Ethylhexyloxyphenol Methoxyphenyl Triazine
Ethylhexyl triazone,
Diethylhexyl Butamido Triazone,
la 2,4,6-tris(4'-amino benzalmalonate de dinéopentyle)-s-triazine
la 2,4,6-tris-(4'-amino benzalmalonate de diisobutyle)-s- triazine
la 2,4bis (4'-aminobenzoate de n-butyle)-6-(aminopropyltrisiloxane)-s-triazine,
la 2,4bis (4'-aminobenzalmalonate de dinéopentyle)-6-(4'-aminobenzoate de n-butyle)-s-triazine,
la 2,4,6-tris(biphenyl-4-yl-1,3,5-triazine
la 2,4,6-tris(terphenyl)-1,3,5-triazine
Drometrizole Trisiloxane

Polysilicone-15

1,1-dicarboxy (2,2'-diméthyl-propyl)-4,4-diphénylbutadiène

2,4-bis-[5-1(diméthylpropyl)benzoxazol-2-yl-(4-phenyl)-imino]-6-(2-ethylhexyl)-imino-1,3,5-triazine

et leurs mélanges.

Les filtres organiques conformes à l'invention représentent en général de 0,1 à 30 %, de préférence de 1 à 25 %, du poids total de la composition.

Les filtres UV inorganiques utilisés conformément à la présente invention sont des pigments d'oxyde métallique. Plus préférentiellement, les filtres UV inorganiques de l'invention sont des particules d'oxyde métallique ayant une taille moyenne de particule élémentaire inférieure ou égale à 500 nm, plus préférentiellement comprise entre 5 nm et 500 nm, et encore plus préférentiellement comprise entre 10 nm et 100 nm, et préférentiellement entre 15 et 50 nm.

Ils peuvent être notamment choisis parmi les oxydes de titane, de zinc, de fer, de zirconium, de cérium ou leurs mélanges.

De tels pigments d'oxydes métalliques, enrobés ou non enrobés sont en particulier décrits dans la demande de brevet EP-A- 0 518 773. A titre de pigments commerciaux on peut mentionner les produits vendus les sociétés Kemira, Tayca, Merck et Degussa.

Les pigments d'oxydes métalliques peuvent être enrobés ou non enrobés.

Les pigments enrobés sont des pigments qui ont subi un ou plusieurs traitements de surface de nature chimique, électronique, mécanochimique et/ou mécanique avec des composés tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :

- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que les produits "MICROTITANIUM DIOXIDE MT 100 T, MT 100 TX, MT 100 Z, MT-01" de la société TAYCA, les produits "Solaveil CT-10 W" et "Solaveil CT 100" de la société UNIQEMA et le produit "Eusolex T-AVO" de la société MERCK,
- de silice, d'alumine et d'acide alginique tel que le produit "MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR 351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA,
- d'alumine et traités par une silicone tels que les produits "TIPAQUE TTO-55 (S)" de la société ISHIHARA, ou "UV TITAN M 262" de la société KEMIRA,
- de triéthanolamine tels que le produit "STT-65-S" de la société TITAN KOGYO,
- d'acide stéarique tels que le produit "TIPAQUE TTO-55 (C)" de la société ISHIHARA, d'hexamétaphosphate de sodium tels que le produit "MICROTITANIUM DIOXIDE MT 150 W" de la société TAYCA.
- le TiO$_2$ traité par l'octyl triméthyl silane vendu sous la dénomination commerciale "T 805" par la société DEGUSSA SILICES,
- le TiO$_2$ traité par un polydiméthylsiloxane vendu sous la dénomination commerciale "70250 Cardre UF TiO2SI3" par la société CARDRE,
- le TiO$_2$ anatase/rutile traité par un polydiméthylhydrogénosiloxane vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple :

- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple :

- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
  ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobés peuvent être par exemple ceux vendus sous la dénomination "COLLOIDAL CERIUM OXIDE" par la société RHONE POULENC.

Les pigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Selon l'invention, les pigments d'oxyde de titane, enrobés ou non enrobés, sont particulièrement préférés.

Les filtres inorganiques conformes à l'invention représentent en général de 0,5 à 40 %, de préférence de 1 à 30 %, du poids total de la composition.

Les compositions aqueuses de l'invention peuvent se présenter sous toutes les formes généralement utilisées pour une application topique, notamment sous forme d'une émulsion huile dans eau (émulsion directe), eau dans huile (émulsion inverse) ou encore d'un gel aqueux.

Les compositions de l'invention peuvent contenir tous les additifs habituellement utilisés en cosmétique et trouveront des applications dans le domaine du Soin, du Maquillage et des produits Solaires.

- Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' «Eldew SL-205 » par la société Ajinomoto), les acides ou les esters gras comme le benzoate d'alcools en $C_{12}$-$C_{15}$ vendu sous la dénomination commerciale « Finsolv TN » ou « Witconol TN » par la société WITCO, le phenylethyl benzoate vendu sous la dénomination XTEND-226 par la société ISP ou SPECTRASOL PEB par la société CP HALL, le palmitate d'octyle, le lanolate d'isopropyle, les triglycérides dont ceux des acides caprique/caprylique, le dicaprylyl carbonate vendu sous la dénomination « Cetiol CC » par la société cognis), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les polymères synthétiques tels que le poly $C_{10}$-$C_{30}$ alkyl acrylate vendu sous la dénomination « Doresco IPA 13-1 » par la société Landec ou encore les argiles modifiées telles que le l'hectorite et ses dérivés, comme les produits commercialisés sous les noms de Bentone.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion, d'un stick. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

- Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

- Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

- Comme esters alkylés de polyol, on peut citer notamment les esters de polyéthylèneglycol comme le PEG-30 Dipolyhydroxystearate tel que le produit commercialisé sous le nom Arlacel P135 par la société ICI.

- Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

- Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

- Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

- Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de soin et/ou de protection solaire pour le visage et/ou le corps de consistance liquide à semi-liquide, telles que des lotions, des laits, des crèmes plus ou moins onctueuses, des gels, des gel-crèmes. Elles peuvent éventuellement être conditionnées en aérosol et se présenter sous forme de mousse ou de spray.

Les compositions cosmétiques selon l'invention peuvent par exemple être utilisées comme produit de maquillage.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les compositions selon l'invention peuvent également comprendre en plus des actifs additionnels cosmétiques et dermatologiques.

Les actifs additionnels pourront notamment être choisis parmi les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, les agents tenseurs, les agents liporestructurants, les agents amincissants, les agents favorisant la microcirculation cutanée, les agents apaisants et/ou anti-irritants, les sébo-régulateurs ou anti-séborrhéiques, les agents astringents, les agents cicatrisants, les agents anti-inflammatoires, et les agents anti-acné.

L'homme du métier choisira le ou lesdits actifs en fonction de l'effet recherché sur la peau, les cheveux, les cils, les sourcils, les ongles.

Pour le soin et/ou le maquillage des peaux âgées, il choisira de préférence au moins un actif choisi parmi les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, , les agents liporestructurants et les agents favorisant la microcirculation cutanée pour le contour des yeux.

La composition pourra comprendre en outre au moins un ingrédient tel que des charges à effet flouteur ou des agents favorisant la coloration naturelle de la peau, destiné à compléter l'effet biologique de ces actifs ou apporter un effet anti-âge immédiat visuel.

Pour le soin et/ou le maquillage des peaux grasses, l'homme du métier choisira de préférence au moins un actif choisi parmi les agents desquamants, agents sébo-régulateurs ou anti-séborrhéiques, les agents astringents.

La composition pourra comprendre en outre au moins un ingrédient additionnel destiné à compléter l'effet biologique de ces actifs ou apporter un effet immédiat visuel ; on peut citer notamment les agents matifiants, les charges à effet flouteur, les agents fluorescents, les agents favorisant al coloration naturellement rosée de la peau et les charges abrasives ou exfoliantes..

1. Adents hydratants ou humectants

Comme agents humectants ou hydratants, on peut citer notamment le glycérol et ses dérivés, l'urée et ses dérivés notamment l'Hydrovance® commercialisée par National Starch, les acides lactiques, l'acide hyaluronique, les AHA, les BHA, le pidolate de sodium, le xylitol, la sérine, le lactate de sodium, l'ectoine et ses dérivés, le chitosane et ses dérivés, le collagène, le plancton, un extrait d'imperata cylindra commercialisé sous la dénomination Moist 24® par la société Sederma, des homopolymères d'acide acrylique comme le Lipidure-HM® de NOF corporation, le beta-glucan et en particulier le sodium carboxymethyl beta-glucane de Mibelle-AG-Biochemistry ; un mélange d'huiles de passiflore, d'abricot, maïs, et son de riz commercialisé par Nestlé sous la dénomination NutraLipids®. ; un dérivé C-glycoside tel que ceux décrits dans la demande WO 02/051828 et en particulier le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® » ; une huile de rosier muscat commercialisée par Nestlé ; un extrait de micro-algue Prophyridium cruentum enrichi en zinc commercialisé par Vincience sous la dénomination Algualane Zinc®. ; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon ; et l'arginine.

De préférence, on utilisera un agent hydratant choisi parmi l'urée et ses dérivés notamment l'Hydrovance® commercialisée par National Starch, l'acide hyaluronique, les AHA, les BHA, des homopolymères d'acide acrylique comme le Lipidure-HM® de NOF corporation, le beta-glucan et en particulier le sodium carboxymethyl beta-glucane de Mibelle-AG-Biochemistry ; un mélange d'huiles de passiflore, d'abricot, maïs, et son de riz commercialisé par Nestlé sous la dénomination NutraLipids® ; un dérivé de C-glycoside tel que ceux décrits dans la demande WO 02/051828 et en particulier le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® » ; une huile de rosier muscat commercialisée par Nestlé ; un extrait de micro-algue Prophyridium cruentum enrichi en zinc commercialisé par Vincience sous la dénomination Algualane Zinc®. ; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon ; et l'arginine.

## 2. Agents desquamants

Par "agent desquamant", on entend tout composé capable d'agir :

- soit directement sur la desquamation en favorisant l'exfoliation, tel que les β-hydroxyacides (BHA), en particulier l'acide salicylique et ses dérivés (dont l'acide n-octanoyl 5-salicylique autrement nommé capryloyl salicylic acid en nom INCI) ; les α-hydroxyacides (AHA), tels que les acides glycolique, citrique, lactique, tartrique, malique ou mandélique ; l'acide 8-hexadécène-1,16-dicarboxylique ou acide 9-octadécène dioïque ; l'urée et ses dérivés; l'acide gentisique et ses dérivés ; les oligofucoses ; l'acide cinnamique ; l'extrait de Saphora japonica ; le resvératrol et certains dérivés d'acide jasmonique ;
  soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques et en particulier l'acide 4-(2-hydroxyethyl)piperazine-1-propanesulfonique (HEPES) ; l'acide 2-oxothiazolidine-4-carboxylique (procystéine) et ses dérivés ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme autres agents desquamants utilisables dans la composition selon l'invention, on peut citer :

- les oligofructoses, l'EDTA et ses dérivés, les laminaria extract, le o-linoleyl-6D-glucose, l'acide (3-hydroxy-2pentylcyclopentyl) acétique, le trilactate de glycérol, l'O-octanyl-6'-D-maltose, la S carboxyméthyl cystéine, les dérivés siliciés de salicylate comme ceux décrits dans le brevet EP 0 796 861, les oligofucase comme ceux décrits dans le brevet EP 0 218 200, les sels d'acide 5-acyl salicylique, des actifs ayant des effets sur la transglutaminase comme dans le brevet EP 0 899 330,
- l'extrait de fleur de ficus opuntia indica comme l'Exfolactive® de Silab,
- l'acide 8-hexadécène 1,16-dicarboxylique,
- les esters de glucose et de vitamine F, et
- leurs mélanges.

Comme agents desquamants préférés, on pourra citer les beta-hydroxyacides, tel que l'acide n-octanoyl 5-salicylique ; l'urée ; les acides glycolique, citrique, lactique, tartrique, malique ou mandélique ; l'acide 4-(2-hydroxyethyl)piperazine-1-propanesulfonique (HEPES) ; l'extrait de Saphora japonica ; le miel ; le N-acétyl glucosamine ;

le méthyl glycine diacétate de sodium, et leurs mélanges.

Encore plus préférentiellement on utilisera dans les compositions de l'invention un agent edsquamant choisi parmi l'acide n-octanoyl 5-salicylique ; l'urée ; l'acide 4-(2-hydroxyethyl)piperazine-1-propanesulfonique (HEPES); l'extrait de Saphora japonica ; le miel ; le N-acétyl glucosamine ; le méthyl glycine diacétate de sodium, et leurs mélanges.

3. Agents améliorant la fonction barrière

Comme agents améliorant la fonction barrière, on peut citer notamment l'arginine, la sérine, un extrait de Thermus thermophilus tel que le Vénucéane® de Sederma, un extrait de rhizome d'igname sauvage (dioscorea villosa) tel que l'Actigen Y® d'Active Organics, des extraits de plancton comme l'oméga plancton® de Secma, des extraits de levure comme le Relipidium® de Coletica, un extrait de chataigne tel que la Recoverine® de Silab, un extrait de bourgeon de cèdre tel que le Gatuline Zen® de Gattefossé, des sphingosines comme la salicyloyl sphingosine vendue sous la dénomination « Phytosphingosine® SLC » par la société Degussa, un mélange de xylitol, de xylityl polyglycoside et de xylitan comme l'Aquaxyl® de Seppic, des extraits de solanacée comme le Lipidessence® de Coletica ; les huiles insaturées en oméga 3 telles que les huiles de rosier muscat et leurs mélanges.

On peut encore citer notamment les céramides ou dérivé, en particulier les céramides de type 2 (comme la N-oléoyldihydrosphingosine ), de type 3 (comme la stearoyl-4-hydroxysphinganine en nom INCI) et de type 5 (comme la N-2-hydroxypalmitoyldihydrosphingosine, ayant pour nom INCI :hydroxypalmytoyl sphinganine), les composés à base de sphingoïdes, les glycosphingolipides, les phospholipides, le cholestérol et ses dérivés, les phytostérols, les acides gras essentiels, le diacylglycérol, la 4-chromanone et dérivés de chromone la vaseline, la lanoline, les beures de karité, le cocoa butter, la lanoline, les sels PCA.

Comme agents préférés ayant un effet restructurant de la barrière cutanée, on citera un extrait de Thermus thermophilus, un extrait de rhizome d'igname sauvage (dioscorea villosa), un extrait de levure, un extrait de chataigne, un extrait de bourgeon de cèdre , l'arginine, la sérine, les céramides notamment de type 3 et 5 ; et leurs mélanges.

De préférence, on utilisera la sérine, l'aginine ou leur mélange.

4. Agents dépigmentants

Comme agents dépigmentants, on pourra citer notamment la vitamine C et ses dérivés et notamment la vit CG, CP et 3-O ethyl vitamine C, l'alpha et la béta arbutine, l'acide férulique, le lucinol et ses dérivés, l'acide kojique, le résorcinol et ses dérivés, l'acide tranexamique et ses dérivés, l'acide gentisique, l'homogentisate, le méthyl gentisate ou l'homogentisate , l'acide dioique, le D calcium panthéteine sulfonate, l'acide lipoique, l'acide ellagique, la vitamine B3, l'acide linoléique et ses dérivés, les céramides et leurs homologues, les dérivés de plantes comme la camomille, la busserole, la famille des aloe (vera, ferox, bardensis), de murier, de scutellaire ; une eau de fruit de kiwi (Actinidia chinensis) commercialisée par Gattefosse, un extrait de racine de Paeonia suffructicosa tel que celui commercialisé par la société Ichimaru Pharcos sous la dénomination Botanpi Liquid B®, un extrait de sucre brun (Saccharum officinarum), tel que l'extrait de melasse commercialisé par la société Taiyo Kagaku sous la dénomination Molasses Liquid, sans que cette liste soit exhaustive.

Comme agents dépigmentants préférés, on utilisera la vitamine C et ses dérivés et notamment la vit CG, CP et 3-O ethyl vitamine C, l'alpha et la béta arbutine, l'acide férulique, l'acide kojique, le résorcinol et ses dérivés, le D calcium panthéteine sulfonate, l'acide lipoique, l'acide ellagique, la vitamine B3, une eau de fruit de kiwi (Actinidia chinensis) commercialisée par Gattefosse, un extrait de racine de Paeonia suffructicosa tel que celui commercialisé par la société Ichimaru Pharcos sous la dénomination Botanpi Liquid B®.

5. Agents antioxvdants

On peut citer notamment le tocophérol et ses esters, en particulier l'acétate de tocophérol ; l'acide ascorbique et ses dérivés, en particulier l'ascorbyl magnésium phosphate et l'ascorbyl glucoside ; l'acide férulique ; la sérine ; l'acide ellagique, la phlorétine, les polyphénols,les tanins, l'acide tannique, l'époigallocathéchines et les extraits naturels en contenant, les anthocyanes, les extraits de romarin, les extraits de feuilles d'olivier comme ceux de la société Silab, les extraits de thé vert, le resvératrol et ses dérivés, l'ergothinéine, la N acétylcystéine, un extrait d'algue brune pelvetia canaliculata comme la Pelvetiane® de Secma, l'acide chlorogénique, la biotine, les chélatants, tels que le BHT, le BHA, le N,N'-bis(3,4,5-triméthoxybenzyl) éthylenediamine et ses sels ; l'idébénone, des extraits végétaux come le Pronalen Bioprotect TM de la société Provital ; le co enzyme Q10, les bioflavonoides, les SOD, le phytantriol, les lignanes, la mélatonine, les pidolates, le gluthation, le caprylyl glycol, la phloretine, le TotaroITM ou extrait de Podocarpus totara contenant du totarol (totara-8, 11, 13-trienol ou 2-phenanthrenol, 4b, 5, 6, 7, 8, 8a, 9, 10-octahydro-4b, 8, 8-trimethyl-1(1-methylethyl)- ; un extrait de jasmin tel que celui commercialisé par SILAB sous la dénomination Helisun® ; le laurate d'hesperitine tel que le Flavagrum PEG® de la société Engelhard Lyon ; un extrait de racine de Paeonia suffructicosa tel que celui commercialisé par la société Ichimaru Pharcos sous la dénomination Botanpi Liquid B® ; un extrait de litchi tel que l'extrait de péricarpe de litchi commercialisé par la société Cognis sous la dénomination Litchiderm LS 9704®, un extrait de fruit de grenade (Punica Granatum), tel que celui commercialisée par la société Draco Natural products.

Comme autre agent anti-âge, on peut citer la DHEA et ses dérivés, l'acide boswellique, les extraits de romarin, les caroténoïdes (B carotène, zéaxanthine, lutéine), l'acide cystéique, les dérivés de cuivre, l'acide jasmonique

Comme agent antioxydant préféré, on utilisera notamment l'acide férulique ; la sérine ; la phlorétine, un extrait de grenade, la biotine, les chélatants, tels que le BHT, le BHA, le N,N'-bis(3,4,5-triméthoxybenzyl) éthylenediamine et ses sels, le caprylyl glycol, la phloretine, le TotarolTM, un extrait de jasmin tel que celui commercialisé par SILAB sous la dénomination Helisun® ; le laurate d'hesperitine tel que le Flavagrum PEG® de la société Engelhard Lyon ; un extrait de racine de Paeonia suffructicosa tel que celui commercialisé par la société Ichimaru Pharcos sous la dénomination Botanpi Liquid B®.

## 6. Agents dermorelaxants ou dermodécontractants

On peut citer comme exemples le gluconate de manganèse et autres sels, l'adénosine, l'alvérine citrate et ses sels, la glycine, un extrait d'Iris pallida, un hexapeptide (Argériline R de Lipotec) ou les sapogénines comme le Wild yarn et les amines carbonylées décrites dans la demande EP1484052. Comme exemple de sapogénines on peut citer celles décrites dans la demande de brevet WO02/47650, en particulier le Wild yarn, la diosgénine extrait notamment de Dioscorea opposita ou tout extrait refermant naturellement ou après traitement une ou plusieurs sapogénines (rhizome d'igname sauvage, feuille d'agave qui contient de l'hécogénine et la tigogénine, extrait de liliacées et plus particulièrement le Yacca ou le smilax contenant la smilagéine et la sarsapogénine, ou la racine de salsepareille) ou l' Actigen Y de la société Actives Organics ; ou le gingembre.

On peut également citer le DMAE (diméthyl MEA), les extraits de criste marine, de ciste de Montpellier, d'hélicryse, d'anis, de Para cress, un extrait d'Acmella Oleracea comme la Gatuline® expression de Gattefossé.

Comme agents dermorelaxants préférés, on citera l'adénosine, le gluconate de manganèse, le wild yarn, la criste marine, la glycine et l'alvérine.

## 7. Agents anti-glycation

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des Ericaceae, tels qu'un extrait de myrtille (Vaccinium angusfifollium, Vaccinium myrtillus), par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE® » par la société SOLABIA, le chorhydrate de carcinine (commercialisé par Exsymol sous la dénomination « ALISTIN®»), un extrait d'Hélianthus annuus comme l'Antiglyskin® de Silab, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® de Laboratoires Sériobiologiques, un extrait de thé noir comme le Kombuchka® de Sederma et leurs mélanges.

Comme agents anti-glycation préférés, on citera les extraits de myrtille (Vaccinium myrtillus) et l'extrait de thé noir.

## 8. Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :

- soit sur la synthèse du collagène, tels que les extraits de Centella asiatica, les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique ; et un extrait de Medicago sativa (alfafa) tel que celui commercialisé par SILBA sous la dénomination Vitanol® ; un extrait peptidique de noisette tel que celui commercialisé par la société Solabia sous la dénomination Nuteline C® ; et l'arginine.

soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi tel que l'extrait de péricarpe de litchi commercialisé par la société Cognis sous la dénomination Litchiderm LS 9704®; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781 ® de Cognis ; l'extrait de sauge décrit dans la demande FR-A-2812544 de la famille des labiées (salvia officinalis de la société Flacksmann), l'extrait de Rhododendron, le blueberry extract, un extrait de vaccinium myrtillus tel que ceux décrits dans la demande FR-A-2814950.

- soit sur la synthèse de molécules appartenent à la famille des élastines (élastine et fibrilline), tels que : le retinol et dérivés en particulier le palmitate de rétinol ; l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ; un extrait peptidique de noisette tel que celui commercialisé par la société Solabia sous la dénomination Nuteline C®.

- soit sur l'inhibition de la dégradation de l'élastine tels que l'extrait peptidique de graines de Pisum sativum commercialisé par la société LSN sous la dénomination commerciale Parelastyl® ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C1-C6. ; un extrait de peptides de riz tel que la Colhibin® de Pentapharm, ou un extrait de Phyllanthus emblica tel que l'Emblica® de Rona.

- soit sur la synthèse des glycosaminoglycannes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin®. ; un extrait de Laminaria ochroleuca tel que la Laminaïne® de Secma ; l'essence de Mamaku de Lucas Meyer, un extrait de cresson (Odraline® de Silab).

- soit sur la synthèse de la fibronectine, tels que l'extrait de zooplancton Salina commercialisé par la société SEPORGA sous la dénomination commerciale GP4G® ;
l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC; et l'extrait de zooplancton Salina commercialisé par la société SEPORGA sous la dénomination commerciale GP4G® ; Tripeptide de Cuivre de PROCYTE ; ; un extrait peptidique de Voandzeia substerranea tel que celui commercialisé par la société Laboratoires Sérobiologiques sous la dénomination commerciale Filladyn LS 9397®.

De préférence on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de Saccharomyces cerevisiae, un extrait de Laminaria ochroleuca, l'essence de Mamaku, un extrait de cresson et leurs mélanges.

Comme actifs préférés stimulant la synthèse de macromolécules dermiques et/ou épidermiques et/ou empêchant leur dégradation, on peut citer :

les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ;; l'acide folique ; un extrait de Medicago sativa (alfafa) tel que celui commercialisé par SILBA sous la dénomination Vitanol® ; un extrait peptidique de noisette tel que celui commercialisé par la société Solabia sous la dénomination Nuteline C® ; l'arginine ; . un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781® de Cognis ; un extrait de vaccinium myrtillus tel que ceux décrits dans la demande FR-A-2814950 ; : le retinol et dérivés en particulier le palmitate de rétinol ; l'extrait de Saccharomyces Cerivisiae commercialisé par la société LSN sous la dénomination commerciale Cytovitin® ; un extrait peptidique de noisette tel que celui commercialisé par la société Solabia sous la dénomination Nuteline C® ; l'acide {2-[acétyl-(3-trifluorométhyl-phényl)-amino]-3-méthyl-butyrylamino acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C1-C6. ; un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin®. ; un extrait de Laminaria ochroleuca tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC.

9. Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.

De préférence on utilisera un agent favorisant la prolifération et/ou la différenciation des kératinocytes.

Les agents stimulant la prolifération des kératinocytes, utilisables dans la composition selon l'invention, comprennent notamment l'adénosine ; le phloroglucinol, l'extrait de feuille d'hydrangea macrophylla comme l'Amacha liquid E® d'Ichimaru Pharcos, un extrait de levure tel que le Stimoderm® de CLR ; l'extrait de Larrea divaricata tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, le phloroglucinol, les extraits de tourteaux de noix commercialisés par Gattefosse et les extraits de solanum tuberosum tel que le Dermolectine® commercialisé par Sederma.

Parmi les agents stimulant la différenciation des kératinocytes comprennent par exemple les minéraux tels que le calcium; la criste marine, un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de retinyl.

Comme agents stimulant la prolifération et/ou la différenciation des kératinocytes, on peut citer encore les oestrogènes tel que l'oestradiol et homologues ; les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C® ; l'adénosine ; le phloroglucinol, un extrait de levure tel que le Stimoderm® de CLR ; un extrait peptidique de lupin tel que celui commercialisé par la société SILAB sous la dénomination commerciale Structurine® ; un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires Sérobiologiques sous la dénomination commerciale Filladyn LS 9397® ; le rétinol et ses esters dont le palmitate de retinyl.

10. Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220 (non publiée).

11. Inhibiteurs de NO-synthases

L'agent ayant une action d'inhibiteur de NO synthase peut être choisi parmi les OPC (oligomères procyanidoliques) ; les extraits de végétal de l'espèce Vitis vinifera notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce Olea europaea de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce Gingko biloba de préférence un extrait aqueux sec de ce végétal vendu par la société Beaufour sous le nom commercial Ginkgo biloba extrait standard et leurs mélanges.

12. Antagonistes des récepteurs périphériques des benzodiazépines (PBR)

On peut citer par exemple le 1-(2-chlorophenyl)-N-(1-methyl-propyl)-3-isoquinoline carboxamide ; les composés décrits dans les demandes WO03/030937, WO03/068753, dérivés de pyridazino[4, 5-b] indole-1-acétamide de formule générale (VII) tels que décrits dans le document WO00/44384.

13. Agents augmentant l'activité de la glande sébacée

On peut citer par exemple le dehydrojasmonate de méthyle, l'hécogénine, l'hédione, le le o-linoleyl-6D-glucose et leurs mélanges.

14. Agents stimulant le métabolisme énergétique des cellules

L'actif stimulant le métabolisme énergétique des cellules peut par exemple être choisi parmi la biotine, un extrait de Saccharomyces cerevisiae tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; un beta-glucan issu de Saccharomyces cerevisiae tel que celui commercialisé par la société Mibelle AG Biochemistry ;

15. Agents tenseurs

Par « agent tenseur » utilisable selon l'invention, on entend des composés susceptibles d'avoir un effet tenseur, c'est-à-dire pouvant tendre la peau.
De manière générale on entend par agent tenseur selon l'invention tous composés solubles ou dispersibles dans l'eau à une température allant de 25°C à 50°C à la concentration de 7% en poids dans l'eau ou à la concentration maximale à laquelle ils forment un milieu d'apparence homogène et produisant à cette concentration de 7% ou à cette concentration maximale dans l'eau une rétractation de plus de 15 % dans le test décrit ci-après.
La concentration maximale à laquelle ils forment un milieu d'apparence homogène est déterminée à $\pm$ 10% près et de préférence à $\pm$ 5% près.
On entend par 'milieu d'apparence homogène' un milieu ne présentant pas d'agrégats visibles à l'oeil nu.
Pour la détermination de ladite concentration maximale, l'agent tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).
L'effet tenseur peut être caractérisé par un test in vitro de rétractation.

On prépare préalablement et tel que décrit précédemment un mélange homogène de l'agent tenseur dans l'eau, à la concentration de 7% en poids ou à la concentration maximale définie précédemment.

30μl du mélange homogène est déposé sur une éprouvette rectangulaire (10x40mm , donc présentant une largeur initiale L0 de 10 mm) d'élastomère ayant un module d'élasticité de 20 MPa et une épaisseur de 100μm.

Après 3h de séchage à 22±3°C et 40±10% d'humidité relative HR, l'éprouvette d'élastomère présente une largeur rétractatée, notée L3h due à la tension exercée par l'agent tenseur déposé.

L'effet tenseur (ET) dudit agent est alors quantifié de la façon suivante :

$$\text{'ET'} = (L0 - L3h / L0) \times 100 \text{ en } \%$$

avec Lo = largeur initiale 10mm
et L3h = largeur après 3h de séchage

L'agent tenseur peut être choisi parmi :

les protéines végétales ou animales et leurs hydrolysats ;
les polysaccharides d'origine naturelle ;
les silicates mixtes ;
les particules colloïdales de charges inorganiques ;
les polymères synthétiques ;

et les mélanges de ceux-ci.

L'homme du métier saura choisir, dans les catégories chimiques listées ci-dessus, les matériaux répondant au test tenseur tel que décrit précédemment.

On peut citer notamment :

(a) les protéines et hydrolysats de protéines végétales, en particulier de maïs, de seigle, de froment, de sarrasin, de sésame, d'épautre, de pois, de fève, de lentille, de soja et de lupin,

(b) les polysaccharides d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,

<u>(c) les silicates mixtes, notamment les phyllosilicates et en particulier les Laponites,</u>

(d) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane. Comme particules colloïdales composites silice-alumine utilisables dans les compositions selon l'invention, on peut citer par exemple celles commercialisées par la société Grace sous les noms de Ludox AM, Ludox AM-X 6021, Ludox HSA et Ludox TMA.

(e) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,

L'agent tenseur sera présent dans la composition en une quantité efficace pour obtenir l'effet biologique recherché selon l'invention.

A titre d'exemple, l'agent tenseur peut être compris dans la composition selon l'invention en une teneur allant de 0,01 à 30% en poids de matière active, de préférence de 1 % à 30% en poids de matière active, par rapport au poids total de la composition.

Par « matière active », on entend exclure le milieu dans lequel l'agent tenseur se trouve éventuellement solubilisé ou en dispersion sous sa forme commerciale, par exemple dans le cas des dispersions de particules colloïdales.

On peut également utiliser, notamment pour complémenter et/ou potentialiser l'effet d'agents tenseurs, utiliser des agents augmentant l'expression des mécanorécepteurs, tels que des agents augmentant l'expression des intégrines. A titre d'exemple, on peut citer un extrait de graine de seigle, tel que celui commercialisé par SILAB sous la dénomination Coheliss©.

### 16. Agents liporestructurants

Par 'agents liporestrucurants', on entend selon l'invention des agents capables de stimuler la lipogénèse et favoriser la différenciation adipocytaire, permettant ainsi d'éviter ou de ralentir la fonte des graisses contenues dans les tissus de soutien de la peau, autrement nommée 'fonte de la lipo-structure de la peau'. Par 'lipo-structure de la peau', on entend le réseau de cellules lipidiques qui forme les volumes sur lesquels la peau du visage repose et se moule.

Ces agents dont destinés à diminuer la perte de densité cutanée et/ou la fonte de la lipo-structure de la peau, en particulier au niveau des joues et du contour de l'oeil, et/ou éviter l'affaissement et/ou le creusement des volumes du visage, la perte de consistance de la peau et/ou son maintien, en particulier au niveau des joues et du contour de l'oeil, et/ouaméliorer les volumes qui sous-tendent la peau du visage et/ou du cou, en particulier au niveau des joues, de l'ovale du visage et du contour de l'oeil, et/ou améliorer la densité, le rebondi et le maintien de la peau, en particulier au niveau des joues, de l'ovale du visage et du contour de l'oeil, et/ourefaçonner les traits du visage, en particulier l'ovale du visage.

Comme exemples d'agents liporestructurants, on peut citer notamment un extrait de thé noir, tel que l'extrait de thé noir fermenté commercialisé par Sederma sous al dénomination Kombuchka®, et un extrait d'Artemisia abrotanum, tel que celui commercialisé par Sllab sous la dénomination Pulpactyl®.

### 17. Agents amincissants

Comme agents amincissants (lipolytiques), on peut citer notamment la caféine, théophylline et ses dérivés, la théobromine, la séricosine, l'acide asiatique, l'acéfylline, l'aminophylline, le chloroéthylthéophylline, le diprofylline, le diniprophylline, l'étamiphylline et ses dérivés, l'étofylline, la proxyphylline ; les extraits de thé, de café, de guarana, de maté, de cola (Cola Nitida) et notamment l'extrait sec de fruit de guarana (Paulina sorbilis) contenant 8 à 10 % de caféine ; les extraits de lierre grimpant (Hedera Helix), d'arnica (Arnica Montana L), de romarin (Rosmarinus officinalis N), de souci (Calendula officinalis), de sauge (Salvia officinalis L), de ginseng (Panax ginseng), de millepertuis (Byperycum Perforatum), de fragon (Ruscus aculeatus L), d'ulmaire (Filipendula ulmaria L), d'orthosiphon (Orthosiphon Stamincus Benth), de bouleau (Betula alba), de cécropia et d'arganier ; les extraits de ginkgo biloba, les extraits de prêle, les extraits d'escine, les extraits de cangzhu, les extraits de chrysanthellum indicum, les extraits de dioscorés riches en diosgénine ou la diosgénine ou hécogénine pure et leurs dérivés, les extraits de Ballote, les extraits de Guioa, de Davallia, de Terminalia, de Barringtonia, de Trema, d'Antirobia, l'extrait de petit grain de bigarrade ; un extrait de coques de fèves de cacao (theobroma cacao) tel que celui commercialisé par Solabia sous la dénomination Caobromine®.

### 18. Agents favorisant la microcirculation cutanée

L'actif agissant sur la microcirculation cutanée peut être utilisé pour éviter le ternissement du teint et/ou améliorer l'aspect du contour de l'oeil, en particulier diminuer les cernes. Il peut être choisi par exemple parmi un extrait d'écorce de pin maritime comme le Pycnogénol® de chez Biolandes, le gluconate de manganèse (Givobio GMn® de Seppic), un extrait d'Ammi visnaga comme la Visnadine d'Indena, l'extrait de lupin (Eclaline® de Silab), le couplage protéine de blé hydrolysée/acide palmitique avec acide palmitique comme l'Epaline 100 des Laboratoires carilène, l'extrait de fleur de bigarade (Remoduline® de Silab), la vitamine P et ses dérivés comme le methyl-4 esculétol mono-éthanoate de sodium vendu sous la dénomination Permethol® par la société Sephytal, les extraits de ruscus, de marron d'inde, de lierre, de ginseng et de mélilot, la caféine, le nicotinate et ses dérivés, la lysine et ses dérivés comme l'Asparlyne® de Solabia, un extrait de thé noir tel que le Kombuchka de Sederma ; les sels de rutine : un extrait d'algue de corallina officinalis tel que celui commercialisé par CODIF ; et leurs mélanges.

Comme agents préférés favorisant la microcirculation cutanée, on citera la caféine, un extrait de fleur de bigarade, un extrait de thé noir, les sels de rutine, un extrait d'algue de corallina officinalis.

### 19. Agents apaisants ou anti-irritants

On entend par agent apaisant un composé permettant de réduire la sensation de picotements, de démangeaisons ou de tiraillements de la peau.

Comme agents apaisants utilisables dans la composition selon l'invention, on peut citer : les oligomères procyannidoliques, les vitamines E, C B5, B3, la caféine et ses dérivés, les triterpènes pentacycliques et les extraits de plantes les contenant, l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra), l'acide oléanolique et ses sels, l'acide ursolique et ses sels, l'acide boswellique et ses sels, l'acide bétulinique et ses sels, un extrait de Paeonia suffruticosa et/ou lactiflora, un extrait de Laminaria saccharina, les extraits de Centella asiatica, l'huile de Canola, le bisabolol, le diesterphosphorique de vitamine E et C comme le Sepivital EPC® de Seppic, les extraits de camomille, l'allantoïne, les huiles insaturées en oméga 3 telles que les huiles de rosier muscat, de cassis, d'Ecchium, de poisson, l'huile de calophilum, des extraits de plancton, la capryloyl glycine, un mélange d'extrait de fleur de nénuphar et de palmitoylproline tel que celui vendu sous la dénomination "Seppicalm VG®" par la société Seppic, un extrait de Boswellia serrata, un extrait de Centipeda cunnighami tel que celui vendu sous la dénomination "Cehami PF®" par la société TRI-K Industries, un extrait de graines de tournesol en particulier l'Hélioxine® de Silab, un extrait de graines de Linum usitatissimum comme la Sensiline® de Silab, les tocotriénols, le piperonal, un extrait d'Epilobium angustifolium tel que celui vendu sous la dénomination "Canadian Willowherb Extract" par la société FYTOKEM PRODUCTS, l'aloe vera, les phytostérols, l'eau de bleuet, l'eau de rose, un extrait de menthe, en particulier de feuilles de menthe comme le Calmiskin® de Silab, les dérivés d'anis, les bactéries filamenteuse comme Vitreoscilla filiformis tel que décrit dans le brevet EP 761 204 et commercialisé par Chimex sous la dénomination Mexoryl SBG®, un extrait de pétales de rose comme le Rose Flower Herbasol® extract de la société Cosmetochem, le beurre de karité, un mélange de fraction de cire de graine d'orge obtenue par CO2 supercritique, de beurre de karité et d'huile d'argan comme le "Stimu-tex AS®" de Pentapharm, les sels alcalino-terreux notamment le strontium, un extrait fermenté d'Alteromonas commercialisé sous la dénomination ABYSSINE® par la société Atrium Biotechnologies ; les eaux thermales du bassin de Vichy, telles que les eaux provenant des sources Célestins, Chomel, Grande-Grille, Hôpital, Lucas et Parc, et de préférence l'eau de la source Lucas ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

Comme agents apaisants préférés selon l'invention, on utilisera :

l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra) ; l'acide ursolique et ses sels ; les extraits de Centella asiatica, l'huile de Canola, le bisabolol ; les extraits de camomille, l'allantoïne ; un mélange d'extrait de fleur de nénuphar et de palmitoylproline tel que celui vendu sous la dénomination "Seppicalm VG®" par la société Seppic ; l'aloe vera, l'eau de rose, un extrait de menthe, en particulier de feuilles de menthe comme le Calmiskin® de Silab, les bactéries filamenteuse comme Vitreoscilla filiformis tel que décrit dans le brevet EP 761 204 et commercialisé par Chimex sous la dénomination Mexoryl SBG®, un extrait de pétales de rose comme le Rose Flower Herbasol® extract de la société Cosmetochem, le beurre de karité, un extrait fermenté d'Alteromonas commercialisé sous la dénomination ABYSSINE® par la société Atrium Biotechnologies ; les eaux thermales du bassin de Vichy, telles que les eaux provenant des sources Célestins, Chomel, Grande-Grille, Hôpital, Lucas et Parc, et de préférence l'eau de la source Lucas ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

20. Agents sébo-régulateurs ou anti-séborrhéiques

Par agents "séborégulateurs ou anti-séborrhéiques ", on entend notamment des agents capables de réguler l'activité des glandes sébacées.

On peut citer notamment :

- l'acide rétinoïque, le peroxyde de benzoyle, le soufre, la vitamine B6 (ou pyridoxine ), le chlorure de sélénium, la criste marine ;
- les mélanges d'extrait de canelle, de thé et d'octanoylglycine tel que le Sepicontrol A5 TEA® de chez Seppic ;
- le mélange de canelle, de sarcosine et d'octanoylglycine, commercialisé notamment par la société SEPPIC sous la dénomination commerciale Sepicontrol A5® ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les dérivés de cuivre et en particulier le pidolate de cuivre tel que Cuivridone® de Solabia ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ; .
- les extraits de reine des prés (spiraea ulamaria) tel que celui vendu sous la dénomination Sébonormine® par la société Silab ;
- les extraits d'algue laminaria saccharina tel que celui vendu sous la dénomination Phlorogine® par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- les extraits de graines de lin tel que celui vendu sous la dénomination Linumine® par la société Lucas Meyer ;
- les extraits de Phellodendron tels que ceux vendu sous la dénomination Phellodendron extract BG par la société Maruzen ou Oubaku liquid B par la société Ichimaru Pharcos ;
- les mélanges d'huile d'argan, d'extrait de serenoa serrulata (saw palmetto) et d'extrait de graines de sésame tel que celui vendu sous la dénomination Regu SEB® par la société Pentapharm ;
- les mélanges d'extraits d'epilobe, de terminalia chebula, de capucine et de zinc biodisponible (microalgues) tel que celui vendu sous la dénomination Seborilys® par la société green tech ;
- les extraits de Pygeum afrianum tel que celui vendu sous la dénomination Pygeum afrianum sterolic lipid extract par la société Euromed ;
- les extraits de serenoa serrulata tels que ceux vendus sous les dénomination Viapure Sabal par la société Actives International, ou ceux vendus par la société Euromed ;
- les mélanges d'extraits de plantain, de berberis aquifolium et de salicylate de sodium tels que celui vendu sous la dénomination Seboclear® par la société Rahn ;
- l'extrait de clou de girofle tel que celui vendu sous la dénomination Clove extract Powder par la société Maruzen ;
- l'huile d'argan telle que celle vendue sous la dénomination Lipofructyl® par les Laboratoires Sérobiologiques ;
- les filtrats de protéine lactique tels que celui vendu sous la dénomination Normaseb® par la société Sederma ;
- les extraits d'algue laminaria, tel que celui vendu sous la dénomination Laminarghane® par la société Biotechmarine ;
- les oligosaccharides d'algue laminaria digitata tel que celui vendu sous la dénomination Phycosaccharide AC par la société Codif ;
- les extraits de sucre de canne tel que celui commercialisé sous la dénomination Policosanol® par la société Sabinsa ;
- l'huile de schiste sulfonée, telle que celle vendue sous la dénomination Ichtyol Pale® par la société Ichthyol ;
- les extraits d'ulmaire (spiraea ulmaria) tels que celui vendu sous la dénomination Cytobiol® Ulmaire par la société Libiol ;
- l'acide sébacique, notamment vendu sous la forme d'un gel de polyacrylate de sodium sous la dénomination Sebosoft® par la société Sederma ;
- les glucomannanes extraits de tubercule de konjac et modifié par des chaînes alkylsulfonates tel que celui vendu sous la dénomination Biopol Beta par la société Arch Chemical ;
- les extraits de Sophora angustifolia, tels que ceux vendus sous la dénomination Sophora powder ou Sophora extract par la société Bioland ;
- les extraits de cinchona succirubra bark tel que celui vendu sous la dénomination le Red bark HS par la société Alban Muller ;
- les extraits de quillaja saponaria tel que celui vendu sous la dénomination Panama wood HS par la société Alban Muller ;
- la glycine greffée sur chaîne undécylénique, telle que celle vendue sous la dénomination Lipacide UG OR par la société Seppic ;
- le mélange d'acide oléanolique et d'acide nordihydroguaïarétique, tel que celui vendus sous la forme d'un gel sous la dénomination AC.Net par la société Sederma ;
- l' acide phthalimidoperoxyhexanoïque ;
- le citrate de trialkyle(C12-C13) vendu sous la dénomination COSMACOL® ECI par la société Sasol ; le citrate de trialkyle(C14-C15) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ; et
- leurs mélanges.

Comme actif anti-séborrhéique préférés, on peut citer :

- le peroxyde de benzoyle, la vitamine B6 (ou pyridoxine ),
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les extraits de reine des prés (spiraea ulamaria) tel que celui vendu sous la dénomination Sébonormine® par la société Silab ;
- les extraits d'algue laminaria saccharina tel que celui vendu sous la dénomination Phlorogine® par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- l'extrait de clou de girofle tel que celui vendu sous la dénomination Clove extract Powder par la société Maruzen ;
- les filtrats de protéine lactique tels que celui vendu sous la dénomination Normaseb® par la société Sederma ;
- les extraits d'ulmaire (spiraea ulmaria) tels que celui vendu sous la dénomination Cytobiol® Ulmaire par la société Libiol ;
- l'acide sébacique, notamment vendu sous la forme d'un gel de polyacrylate de sodium sous la dénomination Sebosoft® par la société Sederma ;
- la glycine greffée sur chaîne undécylénique, telle que celle vendue sous la dénomination Lipacide UG OR par la société Seppic ;
- le citrate de trialkyle(C12-C13) vendu sous la dénomination COSMACOL® ECI par la société Sasol ; le citrate de trialkyle(C14-C15) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ;
- et leurs mélanges.

Préférentiellement, l'actif anti-séborrhéique est choisi parmi :

- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ; et de préférence le pyrrolidone carboxylate de zinc (ou pidolate de zinc) ou le salicylate de zinc ;
- l'extrait de clou de girofle tel que celui vendu sous la dénomination Clove extract Powder par la société Maruzen ;
- la glycine greffée sur chaîne undécylénique, telle que celle vendue sous la dénomination Lipacide UG OR par la société Seppic ;
- le citrate de trialkyle(C12-C13) vendu sous la dénomination COSMACOL® ECI par la société Sasol ; le citrate de trialkyle(C14-C15) vendu sous la dénomination COSMACOL® ECL par la société Sasol ;
- et leurs mélanges.

L'actif anti-séborrhéique est par exemple présent dans une teneur allant de 0,1 à 10 % en poids, de préférence de 0,1 à 5 % en poids, et préférentiellement de 0,5 % à 3 % en poids, par rapport au poids total de la composition.

21. Agents astringents

Par "agents astringents", on entend selon l'invention des agents permettant de lutter contre la dilatation des follicules sébacés.
Comme agents astringents utilisables dans la composition selon l'invention, on peut citer des extraits de pulpe de champignon (polyporus officinalis) comme le "Laricyl LS8865® " de Cognis, des extraits de Terminalia catappa et sambucus nigra comme le Phytofirm LS9120® de Cognis, des extraits de noix de galle comme le Tanlex VE® de Ichimaru Pharcos, l'hydroxychlorure d'aluminium, les extraits de centella (ex Plantactiv centella de Cognis), le chlorure de dicétyl diméthyl ammonium comme le Varisoft 432 CG® de Degussa, les extraits de marron d'inde, les extraits de mauve, les extraits d'Hammamelis, des extraits d'amandes douces, de racines de guimauve et de graines de lin comme l'Almondermin LS 3380® de Cognis, les extraits de bardane, les extraits d'ortie, les extraits de bouleau, les extraits de prêle, les extraits de camomille comme ceux vendus sous la dénomination Extrapone 9 special® par la société Symrise, les extraits de scutellaria, les extrais d'Ulmaire (par exemple le Cytobiol Ulmaire de Libiol), un mélange d'extraits de gingembre blanc, de prêle, d'ortie, de romarin, de yucca comme l'Herb extract B1348® de Bell flavors & fragrances, les extraits d'acacia, d'orme, de saule blanc, de canelle, de bouleau, de reine des prés, les sapogénines de panama, le phenolsulfonate de zinc de Interchemical, des extraits de gentiane, de concombre, de noyer, le mélange d'extraits de Ratanhia, de pamplemousse, de grindelia et de galle de chêne comme l'Epilami® de Alban Muller.
Comme agents astringents préférés selon l'invention, on utilisera les extraits de scutellaria, les extraits d'ulmaire, les extraits de reine des près, les extraits de gentiane, les extraits de bardane et leurs mélanges.

22. Agents cicatrisants

Comme exemples d'agents cicatrisants, on peut citer notamment :

l'allantoine, l'urée, certains acides aminés comme l'hydroxyproline, l'arginine, la sérine, et aussi des extraits de lys blanc (comme le Phytélène Lys 37EG 16295 de Indena), un extrait de levures comme le cicatrisant LS LO/7225B des Laboratoires Sériobiologiques), l'huile de tamanu, l'extrait de saccharomyces cerevisiae comme le Biodynes® TRF® de Arch Chemical, les extraits d'avoine, le chitosane et dérivés comme le glutamate de chitosane, les extraits de carotte, l'extrait d'artemia comme le GP4G® de Vincience, l'acexamate de sodium, des extraits de lavandin, des extraits de propolis, l'acide ximeninique et ses sels, l'huile de rosa rugosa, des extraits de souci comme le Souci Ami® Liposolible d'Alban Muller, des extraits de prêle, les extraits d'écorce de citron comme l'Herbasol® citron de Cosmetochem, des extraits d'helichryse, des extraits de millefeuilles, et l'acide folique.

Comme agents cicatrisants préférés selon l'invention, on utilisera l'arginine, la sérine, l'acide folique, l'huile de tamanu, l'acexamate de sodium, des extraits de prêle, des extraits d'helichryse, et leurs mélanges.

### 23. Agents anti-inflammatoires

Comme agents anti-inflammatoires particuliers utilisables selon l'invention, on peut citer la cortisone, l'hydrocortisone, l'indométhacine, la bétaméthasone, l'acide azéalique, l'acétominophène, le diclofénac, le propionate de clobetasol, l'acide folique ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

Comme agent anti-inflammatoire préféré, on citera l'acide azéalaique, l'acide folique, un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

### 24. Agents anti-acné

Dans un aspect avantageux de l'invention, la composition peut comporter en outre au moins un actif anti-acné.

Par "actif anti-acné", on entend notamment tout actif ayant des effets sur la flore spécifique des peaux grasses tels que par exemple le Propionibacterium acnes (P acnes).

Ces effets peuvent être bactéricides.

A titre d'actifs antibactéricide, on peut citer notamment :

- les actifs et conservateurs à activité anti-microbienne cités dans la demande DE10324567, incorporée dans la présente invention par référence,
- l'acide asiatique,
- le sel de monoéthanolamine du 1-hydroxy-4-methyl 6-trimethylpentyl 2-pyridone (nom INCI: piroctone olamine), notamment vendu sous la dénomination Octopirox® par la société Clariant ;
- l'acide citronellique, l'acide périllique (ou acide 4-isopropenylcyclohex-1-ènecarboxylique ),
- le 2-éthyl hexyle éther de glycérol (nom INCI : ethylhexylglycerine), par exemple vendu sous la dénomination Sensiva SC 50® par la société Shulke & Mayr,
- le caprylate/caprate de glyceryle, par exemple vendu sous la dénomination Capmul MCM® par la société ABITEC ;
- le phosphosilicate de calcium et de sodium, notamment vendu sous les dénominations Bioactive glasspowder® et Actysse Premier BG® par la société Schott Glass ;
- les particules à base d'argent, par exemple celle vendues sous la dénomination Métashine ME 2025 PS® par la société Nippon Sheet Glass ;
- l'extrait de cône de houblon (Humulus Lupulus) obtenu par extraction $CO_2$ supercritique tel que celui vendu sous la dénomination HOP CO2-TO extract® par la société Flavex Naturextrakte,
- l'extrait de Millepertius obtenu par extraction $CO_2$ supercritique tel que celui vendu sous la dénomination ST John's Wort CO2-TO extract® par la société Flavex Naturextrakte,
- le mélange d'extraits de racines de scutellaria baicalensis, de paeonia suffruticosa et de glycyrrhiza glabra, tel que celui vendu sous la dénomination BMB - CF® par la société Naturogin,
- l'extrait d'arganier comme l'Argapure LS9710® de chez COGNIS ;
- les extraits de feuilles de busserole comme celui vendu sous la dénomination "Melfade-J" par la société Pentapharm ;
- l'acide 10-hydroxy-2 décanoique tel que l'Acnacidol P® de chez Vincience, l'ursolate de sodium, l'acide azéalaique, le di-iodo-méthyl p-tolylsulfone tel que l'Amical Flowable® de chez Angus, la poudre de malachite, l'oxyde de zinc tel que Zincare® de chez Elementis GMBH, l'acide octadécènedioïque tel que l'Arlatone dioic DCA® de chez Uniqema ; l'acide ellagique ; le 2,4,4'-trichloro-2'-hydroxy diphényl éther (ou triclosan), la 1-(3',4'-dichlorophényl)-3-(4'-chlorophényl)urée (ou triclocarban), le 3,4,4'-trichlorocarbanilide, le 3',4',5'-trichlorosalicylanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopirox, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octoxyglycérine ou octoglycérine, l'octanoylglycine tel que Lipacid C8G® de Seppic, le caprylyl glycol, l'acide 10-hydroxy-2-décanoïque, le dichlorophenyl imidazol dioxolan et ses dérivés décrits dans la demande de brevet WO9318743, l'iodopropynyl butylcarbamate, le 3,7,11-triméthyldodéca-2,5,10-triénol ou farnesol, les phytosphingosines ; les sels d'ammonium quaternaires comme les sels de cetyltrimethylammonium, les sels de cétylpyridinium, et

- leurs mélanges.

On peut également citer certains tensioactifs ayant un effet antimicrobien comme le cocoampho acétate de sodium ou diacetate disodium tel que le Miranol C2M CONC NP, les bétaines comme la cocoyl bétaine Genagen KB de Clariant, le lauryl ether sulfate de sodium comme l'Emal 270 D de Kao, le décyl glucoside comme le Plantacare 2000 UP, les malate de dialcools C12-13 ramifiés comme le Cosmacol EMI, les monoesters de propylène glycol comme monolaurate, monocaprylate, monocaprate de propylène glycol, le lauryl dimethylamine betaine comme le Empigen BB/LS ainsi que les polyammonium quaternaires comme le Quaternium-24 ou Bardac 2050 de Lonza et ceux décrits dans le brevet FR 0 108 283 et leurs mélanges.

Comme agents antimicrobiens préférés, on utilisera dans les compositions de l'invention un agent choisi parmi l'octoglycérine ou octoxyglycérine, l'acide 10-hydroxy-2-décanoïque, et leurs mélanges.

D'autres actifs anti-acné additionnels peuvent être ajoutés aux actifs anti-acnés précités.

On peut notamment citer les actifs présentant des effets anti-adhésion bactérienne ou bien agissant sur le biofilm des bactéries pour éviter leur multiplication.

Comme agents prévenant et/ou réduisant l'adhésion des micro-organismes, on peut citer notamment : le phytanetriol et ses dérivés tels que décrits dans la demande de brevet EP 1 529 523, les huiles végétales telles de l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia, décrites dans le brevet EP 1 133 979, ou encore certains tensioactifs tels que le cocoamphodiacétate disodique, le cocoate de glycéryle oxyéthyléné (7 OE), l'hexadécénylsuccinate 18, le palmitate d'octoxyglycéryle, le béhénate d'octoxyglycéryle, l'adipate de dioctyle, le PPG-15 stéaryl éther, le tartrate de dialcools C12-C13 ramifiés décrits dans le brevet EP 1 129 694 et leurs mélanges. En particulier vis-à-vis de la propagation du P acnes, ou en tant qu'actifs agissant sur le biofilm des bactéries pour éviter leur prolifération, on peut citer le pentylène glycol, le nylon-66 (fibres de polyamides 66), l'huile de son de riz , l'alcool polyvinylique tel que Celvol 540 PV alcohol® de chez Celanese Chemical, l'huile de colza telle que Akorex L® de chez Karlshamns, et les dérivés de fructose et leurs mélanges.

L'actif agissant contre l'acné peut être présent dans une teneur allant de 0,01 à 10 % en poids, de préférence de 0,05 à 5 % en poids par rapport au poids total de la composition.

En fonction de la nature et/ou de la solubilité des actifs précités, l'homme du métier saura choisir le mode de réalisation le plus adapté selon l'invention.

Comme actifs lipophiles utilisables dans le kit ou l'une au moins des compositions de l'invention, on peut citer notamment le D $\alpha$ tocophérol, le DL $\alpha$ tocophérol, l'acétate de D $\alpha$ tocophérol, l'acétate DL $\alpha$tocophérol, le palmitate d'ascorbyle, les glycérides de vitamine F, les vitamines D, la vitamine D2, la vitamine D3, rétinol, les esters de rétinol, le palmitate de rétinol, le propionate de rétinol, les carotènes dont le $\beta$ carotène, le D panthénol, le farnesol, l'acétate de farnesyle, l'acide salicylique et ses dérivés comme l'acide n-octanoyl-5 salicylique, les alkylesters d' $\alpha$ hydroxyacides tels que l'acide citrique, l'acide lactique, l'acide glycolique, l'acide asiatique, l'acide madécassique, l'asiaticoside, l'extrait total de centella asiatica, l'acide $\beta$ glycyrrhétinique, l'$\alpha$ bisabolol, les céramides comme le 2 oleoylamino-1,3 octadécane, le phytanetriol, les phospholipides d'origine marine riches en acides gras essentiels polyinsaturés, l'éthoxyquine, l'extrait de romarin, l'extrait de mélisse, le quercetine, l'extrait de microalgues séchées, l'huile essentielle de bergamotte, l'octylmethoxycinnamate, le butylméthoxydibenzoylméthane, l'octyl triazone, le di tertiobutyl-3,5 hydroxy-4 benzylidène 3 camphre, les antibiotiques, les antifongiques, les anesthésiques, les analgésiques, les antiseptiques, les antiviraux, les pesticides, les herbicides, et leurs mélanges.

Les actifs cosmétiques et/ou dermatologiques seront présents dans le kit ou l'une des compositions selon l'invention en une teneur allant de 0,001% à 20% en poids par rapport au poids total de la composition, de préférence de 0,01% à 10%, encore plus préférentiellement de 0,5 à 5% et de préférence encore de 0,1 à 1% en poids par rapport au poids total de la composition.

Pour des applications 'peeling', les teneurs en actifs cosmétiques et/ou dermatologiques pourront aller de 1 à 50% en poids par rapport au poids total de la composition, de préférence de 1 à 30% en poids par rapport au poids total de la composition.

Les peelings sont un moyen bien connu pour améliorer l'aspect et/ou la texture de la peau et/ou du cuir chevelu, notamment améliorer l'éclat et l'homogénéité du teint et/ou diminuer les irrégularités visibles et/ou tactiles de la peau, et en particulier pour améliorer l'aspect de surface de la peau, pour atténuer les lentigo actiniques, les marques d'acné ou de varicelle, ainsi que pour prévenir, atténuer ou lutter contres les signes du vieillissement cutané, et notamment pour lisser les irrégularités de la texture de la peau, telles les rides et les ridules.

Ils ont pour effet d'enlever une partie superficielle de la peau à traiter (épiderme et éventuellement couche superficielle du derme), par des méthodes chimiques.

AUTRES INGREDIENTS ADDITIONNELS

Pour complémenter et/ou optimiser les effets conférés par les actifs cosmétiques et/ou dermatologiques cités ci-dessus sur les matières kératiniques, il peut être avantageux d'intégrer dans les compositions de l'invention d'autres ingrédients additionnels.

En particulier, ces ingrédients addditionnels pourront conférer un effet immédiat visuel qui sera relayé par l'effet biologique des actifs cités ci-dessus. Ils pourront également, via une action mécanique (ex : charges abrasvies), amplifier l'effet des actifs biologiqeues cités ci-dessus.

Ainsi la composition selon l'invention pourra comprendre en outre au moins un agent choisi parmi des agents matifiants, des charges à effet flouteur, des agents fluorescents, des agents favorisant la coloration naturellement rosée de la peau, des charges abrasives ou agents exfoliants, et leurs mélanges.

Agents matifiants

Par "agent matifiant", on entend des agents destinés à rendre la peau visiblement plus mate, moins brillante.

L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

L'agent matifiant pourra notamment être choisi parmi un amidon de riz ou un amidon de maïs, la kaolinite, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de polymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les particules de silicate et notamment de silicate mixte, et leurs mélanges.

Comme exemples d'agents matifiants, on peut citer notamment :

- l'amidon de riz ou de maïs, en particulier un aluminium starch octenyl succinate commercialisé sous la dénomination Dry Flo® par la société National Starch,
- la kaolinite ;
- les silices ;
- le talc ;
- un extrait de graines de potiron tel que commercialisé sous la dénomination Curbilene® par la société Indena ;
- des microbilles de cellulose telles que décrites dans la demande de brevet EP 1 562 562 ;
- des fibres, telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742 ;
- des microsphères de copolymères acryliques expansées telles que celles commercialisées par la société EXPANCEL sous les dénominations EXPANCEL 551®,
- des charges à effet optique telles que décrites dans la demande de brevet FR 2 869 796, en particulier :
- les poudres de polyamides (Nylon®), comme par exemple les particules de Nylon 12 du type Orgasol d'Arkema de taille moyenne 10 microns et d'indice de réfraction 1,54,
- les poudres de silice, comme par exemple les Silica beads SB150 de Miyoshi de taille moyenne 5 microns et d'indice de réfraction 1,45,
- les poudres de polytétrafluoroéthylène, comme les PTFE ceridust 9205F de Clariant de taille moyenne 8 microns et d'indice de réfraction 1,36,
- les poudres de résine de silicone comme les Silicon resin Tospearl 145A de GE Silicone de taille moyenne 4,5 microns et d'indice de réfraction 1,41,

- les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100® et F 80 ED® de la société Matsumoto Yushi-Seiyaku,
- les poudres de cire comme les particules Paraffin wax microease 114S de micropowders de taille moyenne 7 microns et d'indice de réfraction 1,54,
- les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
- les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations "KSP-100", "KSP-101", "KSP-102", "KSP-103", "KSP-104", "KSP-105" par la société SHIN ETSU, et
- les poudres composites de talc/dioxyde de titane/alumine/silice comme celles vendues sous la dénomination Coverleaf® AR-80 par la société Catalyst & chemicals,
- leurs mélanges,
- des composés absorbant et/ou adsorbant le sébum tels que décrits dans la demande de brevet FR 2 869 796. On peut citer notamment :
- les poudres de silice, comme par exemple les microsphères de silice poreuses vendues sous la dénomination "SILICA BEADS SB-700" commercialisées par la société MYOSHI, les "SUNSPHERE® H51", "SUNSPHERE® H33" , "SUNSPHERE® H53" commercialisées par la société ASAHI GLASS ; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ;
- les poudres de silicates mixtes amorphes, notamment d'aluminium et de magnésium, comme par exemple celle commercialisée sous la dénomination "NEUSILIN UFL2" par la société Sumitomo.
- les poudres de polyamides (nylon®), comme par exemple "l'ORGASOL® 4000" commercialisé par la société Arkema, et
- les poudres de polymères acryliques, notamment de polyméthacrylate de méthyle, comme par exemple le "COVABEAD® LH85" commercialisé par la société WACKHERR ; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL ; de copolymère diméthacrylate d'éthylène glycol/méthacrylate de lauryle, comme par exemple le "POLYTRAP® 6603" commercialisé de la société DOW CORNING ;
- les particules de silicate, telle que la silicate d'alumine ;
- les particules de silicates mixtes, telles que :
- les particules de silicate d'aluminium et de magnésium, telles que la saponite ou silicate de magnésium et d'aluminium hydraté avec un sulfate de sodium commercialisée sous la dénomination commerciale Sumecton® par la société Kunimine ;
- le complexe silicate de magnésium, hydroxyéthylcellulose, huile de cumin noir, huile de courge et phospholipides ou Matipure® de Lucas Meyer, et
- leurs mélanges.

Comme agents matifiants préférés, on pourra utiliser selon l'invention un extrait de graines de potiron, un amidon de riz ou de mais, la kaolinite, des silices, le talc, les poudres de polyamides, les poudres de polyéthylènes, les poudres de copolymères acryliques, les microsphères de copolymères acryliques expansées, les microbilles de résines de silicones, les particules de silicate mixte et leurs mélanges.

Charges à effet flouteur

Ces charges peuvent être tout matériau susceptible de modifier les rides par ses propriétés physiques intrinsèques et de les masquer. Ces charges peuvent notamment modifier les rides par un effet tenseur, un effet de camouflage, ou un effet de floutage.

En tant que charge, on peut donner à titre d'exemples les composés suivants :

- les microparticules poreuses de silice comme par exemple les Silica Beads® SB 150 et SB 700 de Myochi de taille moyenne de 5μm et les SUNSPHERES® série H d'Asahi Glass comme les H33, H51 de taille respectivement de 3,5 et 5 μm.

- les particules hémisphériques creuses de résines de silicones comme les NLK 500®, NLK 506® et NLK 510® de Takemoto Oil and Fat, notamment décrites dans EP-A-1579849,
- les poudres de résine de silicone comme par exemple les SILICON Resin Tospearl® 145 A DE GE silicone de taille moyenne de 4,5$\mu$m.
- les poudres de copolymères acryliques, notamment de poly(meth)acrylate de méthyle comme par exemple les particules PMMA Jurimer MBI® de Nihon Junyoki de taille moyenne de 8$\mu$m, les sphères creuses de PMMA vendues sous la dénomination COVABEAD® LH 85 par la société Wackherr et les microsphères de vinylidène/acrylonitrile/méthacrylates de méthylène expansées vendues sous la dénomination Expancel®.
- les poudres de cires comme les particules Paraffin wax microloase® 114S de Micropowders de taille moyenne de 7$\mu$m.
- les poudres de polyéthylènes notamment comprenant au moins un copolymère éthylène/acide acrylique comme par exemple les FLOBEADS® EA 209 E de Sumimoto de taille moyenne de 10$\mu$m.
- les poudres d'organopolysiloxanes élastomériques réticulées enrobées de résine de silicone notamment de silsesquioxane sous la dénomination KSP 100®, KSP 101®, KSP 102®, KSP 103®, KSP 104® et KSP 105® par la société Shin Etsu.
- les poudres composites de talc/dioxyde ou de titane/alumine/silice comme par exemple les Coverleaf AR 80® de la société Catalyst & Chemical.
- le talc, le mica, le kaolin, la lauryl glycine, les poudres d'amidon réticulés par l'anhydride octéanyl succinate, le nitrure de bore, les poudres de polytétrafluoroéthylène, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques.
- les fibres hydrophiles ou hydrophobes synthétiques ou naturelles, minérales ou organiques telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de polytétrafluoroéthylène (Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742.
- les silicones réticulés élastomères sphériques comme comme les Trefil E-505C® ou E-506 C® de chez Dow Corning.
- les charges abrasives qui par effet mécanique apportent un lissage du microrelief cutané, telles que la silice abrasive comme par exemple Abrasif SP® de Semanez ou des poudres de noix ou de coques (abricot, noix par exemple de Cosmétochem).

Les charges ayant un effet sur les signes du vieillissement sont notamment choisies parmi des microparticules poreuse de silice, des particules hémisphériques creuses de silicones, des poudres de résine de silicone, des poudres de copolymères acryliques, des poudres de polyéthylènes, des poudres d'organopolysiloxanes élastomériques réticulées enrobées de résine de silicone, des poudres composites de talc/dioxyde de titane/alumine/silice, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques, les fibres de soie, de coton, et leurs mélanges.

La charge peut être une charge « soft focus ».

Par charge « soft-focus », on entend une charge qui en plus donne de la transparence au teint et un effet flou. De préférence, les charges « soft-focus » ont une taille moyenne des particules inférieure ou égale à 15 microns. Ces particules peuvent être de toutes formes et en particulier être sphériques ou non sphériques. De préférence encore, ces charges sont non sphériques.

Les charges « soft-focus » peuvent être choisies parmi les poudres de silice et silicates, notamment d'alumine, les poudres de type polyméthyl méthacrylate (PMMA), le talc, les composites silice/TiO2 ou silice/oxyde de zinc, les poudres de polyéthylène, les poudres d'amidon, les poudres de polyamides, les poudres de copolymères styrène/acrylique, les élastomères de silicone, et leurs mélanges.

En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3® par la société Nippon Talc, la poudre de Nylon® 12, notamment celle vendue sous le dénomination Orgasol 2002 Extra D Nat Cos® par la société Atochem, les particules de silice traitées en surface par une cire minérale 1 à 2 % (nom INCI : hydrated silica (and) paraffin) telles que celles commercialisées par la société Degussa, les microsphères de silice amorphe, telles que celles vendues sous la dénomination Sunsphère par exemple de référence H-53® par la société Asahi Glass, et les micro-billes de silice telles que celles vendues sous la dénomination SB-700® ou SB-150® par la société Miyoshi, cette liste n'étant pas limitative.

La concentration de ces charges ayant un effet sur les signes du vieillissement dans les compositions selon l'invention peut être comprise entre 0,1 et 40 %, voire entre 0,1 et 20 % en poids par rapport au poids total de la composition.

Agents fluorescents

On entend par agent fluorescent une substance qui, sous l'effet de rayons ultraviolet et/ou de la lumière visible, ré-émet dans le visible la portion de lumière qu'elle a absorbé sous la même couleur que celle qu'elle reflète naturellement. La couleur réfléchie naturellement est ainsi renforcée par la couleur ré émise et apparaît extrêmement brillante.

On peut citer par exemple les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

On peut encore citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldéhyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

Les substances fluorescentes sont de préférence présentes dans la composition, à une teneur allant de 0,1 à 20%, de préférence de 0,1 à 15%, de préférence encore de 0,5 à 3% en poids, par rapport au poids total de la composition.

Lorsque les substances fluorescentes organiques sont blanches, on les appelle également des azurants optiques.

L'azurant optique a pour effet d'intensifier l'éclat et aviver les teintes des compositions cosmétiques les comprenant à l'application sur la peau.

Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.

De tels composés sont par exemple disponibles sous les dénominations commerciales Tinopal SOP□et Uvitex OB□auprès de la société CIBA GEIGY.

Les azurants optiques préférentiellement utilisés sont le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate di-sodique et/ou leurs mélanges.

Agents favorisant la coloration naturellement rosée de la peau

On peut citer notamment :

- un agent autobronzant, c'est-à-dire un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV ;
- un agent de coloration additionnel, c'est-à-dire tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage ;

et leurs mélanges.

Comme exemples d'agents autobronzants, on peut citer notamment :

la dihydoxyacétone (DHA),
l'érythrulose, et
l'association d'un système catalytique formé de :

sels et oxydes de manganèse et/ou de zinc, et
hydrogénocarbonates alcalins et/ou alcalinoterreux.

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

D'une manière générale, l'autobronzant est présent en une quantité allant de 0,01 à 20 % en poids, et de préférence en quantité comprise entre 0,1 et 10 % du poids total de la composition.

On peut encore utiliser d'autres colorants qui permettent de modifier la couleur produite par l'agent autobronzant.

Ces colorants peuvent être choisis parmi les colorants directs synthétiques ou naturels.

Ces colorants peuvent être choisis par exemple parmi les colorants rouges ou oranges du type fluorane tels que ceux décrits dans la demande de brevet FR2840806. On peut citer par exemple les colorants suivants :

- le tetrabromofluoroscéine ou éosine connue sous le nom CTFA : CI 45380 ou Red 21
- la phloxine B connue sous le nom CTFA : CI 45410 ou Red 27
- la diiodofluorescéine connue sous le nom CTFA CI 45425 ou Orange 10 ;
- la dibromofluorescéine connue sous le nom CTFA : CI 45370 ou Orange 5.
- le sel de sodium de la tetrabromofluoroscéine connue sous le nom CTFA : CI 45380 (Na salt) ou Red 22
- le sel de sodium de la phloxine B connu sous le nom CTFA : CI 45410 (Na salt) ou Red 28
- le sel de sodium de la diiodofluorescéine connu sous le nom CTFA : CI 45425 (Na salt) ou Orange 11 ;
- l'érythrosine connu sous le nom CTFA : CI 45430 ou Acid Red 51.
- la phloxine connu sous le nom CTFA : CI 45405 ou Acid Red 98.

Ces colorants peuvent être également choisis parmi les antraquinones , le caramel, le carmin, le noir de charbon, les bleus azulènes, le methoxalène, le trioxalène, le guajazulène, le chamuzulène, le rose de bengale, la cosine 10B, la cyanosine, la daphinine.

Ces colorants peuvent être également choisis parmi les dérivés indoliques comme les monohydroxyindoles tels que décrits dans le brevet FR2651126 ( ie : 4-, 5-, 6-ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole) ;

Charges abrasives ou agents exfoliants

Comme agents exfoliants utilisables dans des compositions rincées selon l'invention, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges.

On peut citer aussi l'Exfogreen® de Solabia (extrait de bambou), des extraits d'akenes de fraises (Akenes de fraise de Greentech), de la poudre de noyau de pêche, la poudre de noyau d'abricot, et enfin dans le domaine des poudres végétales à effet abrasif, citons la poudre de noyaux d'airelles (cranberry). Comme charges abrasives ou agents exfoliants préférés selon l'invention, on citera la poudre de noyaux de pêche, la poudre de noyaux d'abricot, la poudre de noyaux d'airelles, les extraits d'akènes de fraise, les extraits de bambou.

Les exemples qui suivent servent à illustrer l'invention sans toutefois présenter un caractère limitatif. Dans ces exemples, les quantités des ingrédients compositions sont données en % pondéral par rapport au poids total de la composition.

**1) Exemples de fabrication de polymères semi-cristallins**

**Exemple 1 : Polymère acide de point de fusion de 40 °C**

Dans un réacteur d'1l muni d'une agitation centrale avec ancre, d'un réfrigérant et d'un thermomètre, on introduit 120 g d'huile de Parléam® (huile minérale) que l'on chauffe de la température ambiante à 80°C en 45 min. A 80°C, on introduit en 2h le mélange $C_1$ suivant :

40 g de cyclohexane + 4 g de Triganox 141 [ 2,5 bis (2-éthyl hexanoyl peroxy) - 2,5 - diméthyl hexane].
30 min après le début de la coulée du mélange $C_1$, on introduit en 1h30 le mélange $C_2$ constitué de :

190 g d'acrylate de stéaryle + 10 g d'acide acrylique + 400 g de cyclohexane.

A la fin des deux coulées, on laisse réagir 3h supplémentaires à 80°C puis on distille à pression atmosphérique la totalité du cyclohexane présent dans le milieu réactionnel.

On obtient alors le polymère à 60 % en poids en matière active dans l'huile de Parléam®.

Sa masse moléculaire moyenne en poids $M_w$ est de 35 000 exprimée en équivalent polystyrène et sa température de fusion pF est de 40°C $\pm$ 1°C, mesurée par D.S.C.

### Exemple 2 : Polymère basique de point de fusion de 38 °C

On applique le même mode opératoire que dans l'exemple 1, sauf que l'on utilise de la N-Vinyl Pyrrolidone en lieu et place de l'acide acrylique.

Le polymère obtenu est à 60% en poids en matière active dans l'huile de Parléam®, sa masse moléculaire moyenne en poids $M_w$ est de 38 000 et son pF de 38°C.

### Exemple 3 : Polymère Acide de point de fusion de 60 °C

On applique le même mode opératoire que dans l'exemple 1, sauf que l'on utilise de l'acrylate de béhényle en lieu et place de l'acrylate de stéaryle. Le polymère obtenu est à 60% en poids en matière active dans l'huile de Parléam®. Sa masse moléculaire moyenne en poids $M_w$ est de 42 000 et son pF de 60°C.

### Exemple 4 : Polymère basique de point de fusion de 58 °C

On applique le même mode opératoire que dans l'exemple 2, sauf que l'on utilise de l'acrylate de béhényle en lieu et place de l'acrylate de stéaryle. Le polymère obtenu est à 60% en poids en matière active dans l'huile de Parléam®. Son $M_w$ est de 45 000 et son pF est de 58°C.

### II) Exemples 1 à 4 de composition

| Ingrédients | Ex 1 | Ex 2 * | EX 3 * | EX 4 * |
|---|---|---|---|---|
| Phase $A_1$ | | | | |
| EDTA disodique | 0,1 | 0,1 | 0,1 | 0,1 |
| Conservateur | 1,25 | 1,25 | 1,25 | 1,25 |
| Glycérine | 4 | 4 | 4 | 4 |
| Propyleneglycol | 4 | 4 | 4 | 4 |
| Eau | 61,65 | 61,65 | 61,65 | 61,65 |
| Phase $A_2$ | | | | |
| Dispersion de particules à 86% en poids Styrene/Acrylates Copolymer (Sunspheres Powder de ROHM & HAAS) | 2 | 2 | - | - |
| Phase $B_1$ | | | | |
| Poly($C_{10}$-$C_{30}$)alkylacrylates (Intelimer 13-1 de LANDEC) | 1 | - | 1 | - |
| Mélange de séarate de glycéryle, alcool béhénique, sodium sulfate de dicocoylethylendiamine PEG-15, stéarate citrate de glycéryle | 2 | 2 | 2 | 2 |
| Isononyl isononaoate | 4 | 4 | 4 | 4 |
| Alcool cétylique | 0,5 | 0,5 | 0,5 | 0,5 |
| Butylmethoxy Dibenzoylmethane | 3 | 3 | 3 | 3 |
| Ethylhexyl Salicylate | 5 | 5 | 5 | 5 |
| Octocrylene | 7 | 7 | 7 | 7 |

(suite)

| Ingrédients | Ex 1 | Ex 2 * | EX 3 * | EX 4 * |
|---|---|---|---|---|
| Phase B$_2$ | | | | |
| Parfum | 0,4 | 0,4 | 0,4 | 0,4 |
| Tocophérol | 0,2 | 0,2 | 0,2 | 0,2 |
| Acrylates Copolymer | 0,7 | 0,7 | 0,7 | 0,7 |
| Phase D | | | | |
| Triéthanolamine | 0,2 | 0,2 | 0,2 | 0,2 |
| • * hors invention | | | | |

On mesure pour chacune des compositions 1 à 4 le SPF in vivo moyen sur 5 volontaires selon la méthode internationale du COLIPA de 2003

Les résultats obtenus sont indiqués dans le tableau suivant :

| | Ex1 | EX2* | EX3* | EX4* |
|---|---|---|---|---|
| Nombre de sujets | 5 | 5 | 5 | 5 |
| SPF moyen in vivo | 29,5 | 11,8 | 9,8 | 4,8 |
| • * hors invention | | | | |

On observe que, dans la composition 1 selon l'invention, l'association polymère semi-cristallin/particules de latex creuses conduit à une synergie au niveau du SPF par rapport à chacun des composants utilisé seul respectivement dans la composition 2 et 3..

Le SPF moyen de la composition 1 (29,5) est supérieur de 35% à la somme (21,6) des SPF moyens des compositions 2 et 3.

Le SPF moyen de la composition 1 (29,5) est 6 fois supérieur au SPF de la composition 4 (4,8) ne contenant aucun des composant de l'association

## Revendications

1. Composition cosmétique ou dermatologique comprenant au moins un filtre UV organique et/ou un filtre inorganique, **caractérisée par le fait qu'**elle contient, dans un milieu physiologiquement acceptable au moins l'association (A) et (B) suivante :

    A) un polymère semi-cristallin solide à température ambiante et ayant une température de fusion supérieure ou égale à 30°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre Mn supérieure ou égale à 1000 ; ledit polymère étant choisi parmi les polymères issus d'un **monomère à chaîne cristallisable choisi parmi les (méth)acrylates d'alkyle saturés en C$_{14}$ à C$_{22}$ et**
    B) des particules de latex creuses **constituées d'un copolymère de styrène et d'acide (méth)acrylique ou l'un de ses (C$_1$-C$_{20}$)alkyl esters** ayant une taille de particule allant de 150 à 380 nm.

2. Composition selon la revendication 1, **caractérisée par le fait que** le polymère semi-cristallin a un point de fusion allant de 30 °C à 80 °C, de préférence allant de 30 °C à 70 °C.

3. Composition selon l'une des revendications précédentes, dans laquelle le polymère semi-cristallin présente une masse moléculaire moyenne en nombre Mn allant de 2 000 à 800 000.

4. Composition **selon l'une quelconque des revendications précédentes, caractérisée en ce que** le polymère semi-cristallin est choisi parmi les poly(acrylate de stéaryle) ou les poly(acrylate de béhényle).

5. Composition selon l'une des revendications précédentes, **caractérisée en ce que** le polymère semi-cristallin est présent en une teneur allant de 0,1 à 50 % en poids, par rapport au poids total de la composition, et mieux allant de 0,5 à 20 % en poids, et encore mieux allant de 1 % à 10 % en poids.

6. Composition selon l'une des revendications précédentes, **caractérisée en ce que** les particules creuses de latex ont une taille de particule allant de 150 à 375 nm et plus préférentiellement de 190 à 350 nm et plus particulièrement de 251 à 325 nm.

7. Composition selon l'une quelconque des revendications 1 à **6**, où les particules de latex sont obtenues à partir de particules comprenant au moins un polymère pour le coeur et au moins un polymère pour l'enveloppe.

8. Composition selon l'une quelconque des revendications précédentes, où les particules creuses de latex sont présentes dans des quantités allant de 0,1 à 20% en poids et plus préférentiellement de 0,5 à 10% en poids par rapport au poids total de la composition.

9. Composition selon l'une quelconque des revendications précédentes, où le ou les filtres UV organiques sont choisis parmi les dérivés cinnamiques ; les anthranilates ; les dérivés salicyliques, les dérivés de dibenzoylméthane, les dérivés du camphre; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes ; les dérivés de mérocyanine et leurs mélanges.

10. Composition selon l'une quelconque des revendications précédentes où le ou les filtres inorganiques sont des pigments d'oxyde de titane, de zinc, de fer, de zirconium, de cérium, enrobés ou pas ou leurs mélanges ayant une taille moyenne de particule élémentaire inférieure ou égale à 500 nm, plus préférentiellement comprise entre 5 nm et 500 nm, et encore plus préférentiellement comprise entre 10 nm et 100 nm, et préférentiellement entre 15 et 50 nm.

11. Utilisation cosmétique de l'association de (A) un polymère semi-cristallin et (B) des particules creuses de latex telle que définie dans les revendications précédentes, dans une composition à application topique destinée à la protection de la peau, des lèvres, des ongles, des cheveux, du cuir chevelu, des cils ou des sourcils, ongles contre les rayonnements UV, en particulier le rayonnement solaire, contenant au moins un filtre UV organique et/ou un filtre UV minéral, comme agent permettant d'augmenter le facteur de protection solaire (SPF).

**Patentansprüche**

1. Kosmetische oder dermatologische Zusammensetzung, umfassend mindestens einen organischen UV-Filter und/oder einen anorganischen Filter, **dadurch gekennzeichnet, dass** sie in einem physiologisch unbedenklichen Medium mindestens die folgende Kombination (A) und (B) enthält:

   A) ein teilkristallines Polymer, das bei Umgebungstemperatur fest ist und eine Schmelztemperatur größer gleich 30°C aufweist, mit a) einer Polymerhauptkette und b) mindestens einer kristallisierbaren organischen Seitenkette und/oder einem kristallisierbaren organischen Block, der Teil der Hauptkette des Polymers ist, wobei das Polymer eine zahlenmittlere Molmasse Mn größer gleich 1000 aufweist; wobei das Polymer aus Polymeren aus einem Monomer mit kristallisierbarer Kette, das aus gesättigten $C_{14}$- bis $C_{22}$-Alkyl(meth)-acrylaten ausgewählt ist, ausgewählt ist; und
   B) hohle Latexteilchen, die aus einem Copolymer von Styrol und (Meth)acrylsäure oder einem ihrer ($C_1$-$C_{20}$)-Alkylester aufgebaut sind und eine Teilchengröße im Bereich von 150 bis 380 nm aufweisen.

2. Zusammensetzung nach Anspruch 1, **dadurch gekennzeichnet, dass** das teilkristalline Polymer einen Schmelzpunkt im Bereich von 30°C bis 80°C und vorzugsweise im Bereich von 30°C bis 70°C aufweist.

3. Zusammensetzung nach einem der vorhergehenden Ansprüche, wobei das teilkristalline Polymer eine zahlenmittlere Molmasse Mn im Bereich von 2000 bis 800.000 aufweist.

4. Zusammensetzung nach einem der vorhergehenden Ansprüche, **dadurch gekennzeichnet, dass** das teilkristalline Polymer aus Poly(stearylacrylat)en und Poly(behenylacrylat)en ausgewählt ist.

**EP 2 011 481 B1**

5. Zusammensetzung nach einem der vorhergehenden Ansprüche, **dadurch gekennzeichnet, dass** das teilkristalline Polymer in einer Menge im Bereich von 0,1 bis 50 Gew.-%, bezogen auf das Gesamtgewicht der Zusammensetzung, besser im Bereich von 0,5 bis 20 Gew.-% und noch besser im Bereich von 1 bis 10 Gew.-% vorliegt.

6. Zusammensetzung nach einem der vorhergehenden Ansprüche, **dadurch gekennzeichnet, dass** die hohlen Latexteilchen eine Teilchengröße im Bereich von 150 bis 375 nm und weiter bevorzugt von 190 bis 350 nm und spezieller von 251 bis 325 nm aufweisen.

7. Zusammensetzung nach einem der Ansprüche 1 bis 6, wobei die Latexteilchen aus Teilchen, die mindestens ein Polymer für den Kern und mindestens ein Polymer für die Hülle umfassen, erhalten werden.

8. Zusammensetzung nach einem der vorhergehenden Ansprüche, wobei die hohlen Latexteilchen in Mengen im Bereich von 0,1 bis 20 Gew.-% und weiter bevorzugt 0,5 bis 10 Gew.-%, bezogen auf das Gesamtgewicht der Zusammensetzung, vorliegen.

9. Zusammensetzung nach einem der vorhergehenden Ansprüche, wobei der organische UV-Filter bzw. die organischen UV-Filter aus Zimtsäurederivaten, Anthranilaten; Salicylsäurederivaten, Dibenzoylmethanderivaten, Campherderivaten; Benzophenonderivaten; β,β-Diphenylacrylatderivaten; Triazinderivaten; Benzotriazolderivaten; Benzalmalonatderivaten; Benzimidazolderivaten; Imidazolinen; Bisbenzoazolylderivaten; p-Aminobenzoesäurederivaten (PABA-Derivaten); Methylenbis(hydroxyphenylbenzotriazol)derivaten; Benzoxazolderivaten; Filterpolymeren und Filtersilikonen; von α-Alkylstyrol abgeleiteten Dimeren; 4,4-Diarylbutadienen; Merocyaninderivaten und Mischungen davon ausgewählt ist bzw. sind.

10. Zusammensetzung nach einem der vorhergehenden Ansprüche, wobei es sich bei dem anorganischen Filter bzw. den anorganischen Filtern um beschichtete oder unbeschichtete Pigmente aus Titan-, Zink-, Eisen-, Zirconium- und Ceroxid oder Mischungen davon mit einer mittleren Elementarteilchengröße kleiner gleich 500 nm, weiter bevorzugt zwischen 5 nm und 500 nm und noch weiter bevorzugt zwischen 10 nm und 100 nm und vorzugsweise zwischen 15 und 50 nm handelt.

11. Kosmetische Verwendung der Kombination von (A) einem teilkristallinen Polymer und (B) hohlen Latexteilchen gemäß den vorhergehenden Ansprüchen in einer Zusammensetzung zur topischen Anwendung, die für den Schutz der Haut, der Lippen, der Nägel, der Haare, der Kopfhaut, der Wimpern, der Augenbrauen gegen UV-Strahlung, insbesondere Sonnenstrahlung, bestimmt ist und mindestens einen organischen UV-Filter und/oder einen anorganischen UV-Filter als Mittel, das die Erhöhung des Lichtschutzfaktors (LSF) erlaubt, enthält.

**Claims**

1. Cosmetic or dermatological composition comprising at least one organic UV screening agent and/or one inorganic screening agent, **characterized in that** the said composition comprises in a physiologically acceptable medium at least the following combination (A) and (B):

   A) a semi-crystalline polymer which is solid at ambient temperature and has a melting point of greater than or equal to 30°C, containing a) a polymeric backbone and b) at least one crystallizable organic side chain and/or one crystallizable organic block forming part of the backbone of the said polymer, the said polymer having a number-average molecular mass Mn of greater than or equal to 1000; the said polymer being selected from polymers obtained from a monomer having a crystallizable chain and selected from saturated $C_{14}$ to $C_{22}$ alkyl (meth)acrylates, and
   B) hollow latex particles constituted of a copolymer of styrene and (meth)acrylic acid or one of its $C_1$-$C_{20}$ alkyl esters and having a particle size ranging from 150 to 380 nm.

2. Composition according to Claim 1, **characterized in that** the semi-crystalline polymer has a melting point ranging from 30°C to 80°C, preferably ranging from 30°C to 70°C.

3. Composition according to either of the preceding claims, wherein the semi-crystalline polymer has a number-average molecular mass Mn ranging from 2000 to 800 000.

4. Composition according to any one of the preceding claims, **characterized in that** the semi-crystalline polymer is

34

selected from poly(stearyl acrylate)s and poly(behenyl acrylate)s.

5.  Composition according to one of the preceding claims, **characterized in that** the semi-crystalline polymer is present in an amount ranging from 0.1% to 50% by weight, relative to the total weight of the composition, and better still ranging from 0.5% to 20% by weight, and even better still ranging from 1% to 10% by weight.

6.  Composition according to one of the preceding claims, **characterized in that** the hollow latex particles have a particle size ranging from 150 to 375 nm and more preferably from 190 to 350 nm and more particularly from 251 to 325 nm.

7.  Composition according to any one of Claims 1 to 6, where the latex particles are obtained from particles comprising at least one polymer for the core and at least one polymer for the shell.

8.  Composition according to any one of the preceding claims, where the hollow latex particles are present in amounts ranging from 0.1% to 20% by weight and more preferably from 0.5% to 10% by weight relative to the total weight of the composition.

9.  Composition according to any one of the preceding claims, where the organic UV screening agent or agents are selected from cinnamic derivatives; anthranilates; salicylic derivatives; dibenzoylmethane derivatives; camphor derivatives; benzophenone derivatives; $\beta,\beta$-diphenylacrylate derivatives; triazine derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; $\alpha$-alkylstyrene-derived dimers; 4,4-diarylbutadienes; merocyanin derivatives; and mixtures thereof.

10. Composition according to any one of the preceding claims, where the inorganic screening agent or agents are coated or uncoated oxide pigments of titanium, zinc, iron, zirconium, cerium, or their mixtures having an average elementary particle size of less than or equal to 500 nm, more preferably between 5 nm and 500 nm and more preferably still between 10 nm and 100 nm, and preferably between 15 and 50 nm.

11. Cosmetic use of the combination of (A) a semi-crystalline polymer and (B) hollow latex particles, as defined in the preceding claims, in a composition for topical application which is intended to protect the skin, lips, nails, hair, scalp, lashes or brows against UV radiation, more particularly solar radiation, and comprises at least one organic UV screening agent and/or one inorganic UV screening agent, as an agent allowing the sun protection factor (SPF) to be increased.

**EP 2 011 481 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5663213 A
- EP 11092421 A
- EP 1281388 A
- EP 1281389 A
- EP 1291390 A
- DE 10138499
- EP 1331000 A
- US 4427836 A
- US 4469825 A
- US 4594363 A
- US 4677003 A
- US 4920160 A
- US 4970241 A
- EP 267726 A
- EP 331421 A
- US 490229 A
- US 5157084 A
- EP 1092421 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 04006878 A
- WO 05058269 A
- WO 06032741 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0518773 A
- WO 9206778 A
- FR 2315991
- FR 2416008
- US 4077441 A
- US 4850517 A
- WO 02051828 A
- EP 0852949 A
- EP 0796861 A
- EP 0218200 A
- EP 0899330 A
- EP 1484052 A
- WO 0247650 A
- FR 2802425
- FR 2810548
- FR 2796278
- FR 2802420
- FR 2812544 A
- FR 2814950 A
- WO 0194381 A
- FR 2877220
- WO 03030937 A
- WO 03068753 A
- WO 0044384 A
- EP 1038519 A
- EP 761204 A
- DE 10324567
- WO 9318743 A
- FR 0108283
- EP 1529523 A
- EP 1133979 A
- EP 1129694 A
- EP 1562562 A
- EP 1151742 A
- FR 2869796
- US 5538793 A
- EP 1579849 A
- FR 2466492
- WO 9735842 A
- EP 903342 A
- WO 9725970 A
- FR 2840806
- FR 2651126
- EP 0425324 B

**Littérature non-brevet citée dans la description**

- Symetrical Triazine Derivatives. IP.COM Journal. IP.COM INC WEST HENRIETTA, 20 Septembre 2004

- **BANGHAM ; STANDISH ; WATKINS.** *J. Mol. Biol.,* 1965, vol. 13, 238